# FASCICULE DE BREVET EUROPEEN

(11) **EP 2 588 599 B1**
(45) Date de publication et mention de la délivrance du brevet: **30.11.2016**
(21) Numéro de dépôt: 11729114.6
(22) Date de dépôt: 01.07.2011
(51) Int. Cl.: C12N 9/02, C12N 9/90, C12P 7/64

(54) **OPTIMISATION DE LA SYNTHESE ET DE L'ACCUMULATION DE LIPIDES**
OPTIMIERUNG DER SYNTHESE UND DER AKKUMULIERUNG VON LIPIDEN
IMPROVMENT CONCERNING THE SYNTHESIS AND THE ACCUMULATION OF LIPIDES

(30) Priorité: 01.07.2010 FR 1055331
(43) Date de publication de la demande: 08.05.2013
(73) Titulaire: INSTITUT NATIONAL DE LA RECHERCHE AGRONOMIQUE, 75007 Paris (FR)
(72) Inventeur: DULERMO, Thierry, 78100 Saint Germain En Laye (FR); NICAUD, Jean-Marc, 78190 Trappes (FR)
(74) Mandataire: Regimbeau
(86) Numéro de dépôt international: PCT/EP2011/061106
(87) Numéro de publication internationale: WO 2012/001144

(56) Documents cités:
- WO-A2-2005/118814
- WO-A2-2010/004141
- BEOPOULOS ATHANASIOS ET AL: "Control of Lipid Accumulation in the Yeast Yarrowia lipolytica", APPLIED AND ENVIRONMENTAL MICROBIOLOGY, vol. 74, no. 24, décembre 2008 (2008-12), pages 7779-7789, XP002620271, ISSN: 0099-2240 cité dans la demande
- FRANCE THEVENIEAU ET AL: 'Microorganisms as sources of oils' OCL vol. 20, no. 6, D603, 01 Novembre 2013, pages 1 - 8, XP055096111 DOI: 10.1051/ocl/2013034 ISSN: 2257-6614

## Description

Plusieurs technologies telles que la fermentation à grande échelle sont appliquées pour la production industrielle d'huile à partir de microorganismes en utilisant comme substrat des matières grasses ou du glycérol. Dans le cadre de ces projets, les microorganismes sont utilisés comme usine cellulaire en réorientant leur métabolisme cellulaire vers la production de composés d'intérêt industriel ou alimentaire, tels que les esters cireux, les isoprénoides, les polyhydroxyalkanoates et les acides gras hydroxylés. La plupart de ces technologies ciblent la production de lipides de réserve avec une structure et/ou une composition spécifique. Ceux-ci comprennent les huiles enrichies en acides gras polyinsaturés essentiels qui peuvent être potentiellement utilisés comme complément alimentaire, les lipides ayant des similitudes de composition au beurre de cacao et des huiles non spécifiques destinées à être utilisées dans la synthèse des biocarburants.

De ce fait, on constate un intérêt grandissant pour l'amélioration de la composition et de la teneur en huile des microorganismes, particulièrement des levures.

Les microorganismes oléagineux sont des organismes capables d'accumuler des réserves lipidiques pouvant dépasser 20 % de la matière sèche de la cellule. La levure *Yarrowia lipolytica* est, parmi ces microorganismes, l'un des plus étudiés et les plus utilisés, de par sa capacité à accumuler des lipides cellulaires jusqu'à 40 % de son poids sec.

Les levures, et en particulier *Y. lipolytica,* sont capables d'utiliser efficacement des substrats hydrophobes, par exemple des alcanes, des acides gras et des huiles comme seule source de carbone (Fickers et al., FEMS Yeast Research, 5(6-7): 527-543, 2005). Les chaînes aliphatiques ingérées peuvent être utilisées pour la production d'énergie ou accumulées sous des formes inchangées ou modifiées.

Les molécules de stockage telles que les triglycérides (TG) et/ou les esters de stérols, (sterytesters ; SE), incapables de s'intégrer dans les bicouches de phospholipides, se groupent pour former le noyau hydrophobe de corps lipidiques (lipid bodies ; LB).

Lesdits corps lipidiques ont longtemps été uniquement considérés comme un stockage de lipides neutres pouvant être mobilisés en période de privation. Toutefois, l'image desdits corps lipidiques comme simple compartiment de stockage a dû être révisée depuis que de nombreuses protéines desdits corps lipidiques ont été identifiées comme des enzymes impliquées dans le métabolisme lipidique, en particulier dans la synthèse et/ou la dégradation des triglycérides.

Dans les levures, la synthèse des triglycérides suit la voie Kennedy. Les acides gras libres sont activés pour le coenzyme A (CoA) et utilisés pour l'acylation du glycérol, pivot de la synthèse des triglycérides.

Dans la première étape d'assemblage de triglycérides, le glycérol-3-phosphate (G-3-P) est acylé par l'acyltransférase spécifique du glycérol-3-phosphate (glycerol-3-phosphate acyltransférase ou *SCT1*) pour donner de l'acide lysophospatidique, qui est ensuite acylé par l'acyltransférase spécifique de l'acide lysophosphatidique (Phosphatidic acid acyltransférase ou *SLC1*) pour donner de l'acide phosphatidique (PA). Celui-ci est ensuite déphosphorylé par une phosphohydrolase spécifique de l'acide phosphatidique (phosphatidic acid phosphohydrolase (PAP)) pour libérer du diacylglycérol (DAG).

Dans la dernière étape le diacylglycérol est acylé soit par une diacylglycérol acyltransférase soit par une phospholipide diacylglycérol acyltransférase pour produire des triglycérides.

Le tableau 1 décrit les gènes impliqués dans le métabolisme des acides gras chez les levures, particulièrement chez *Yarrowia lipolytica*, (YL). Les séquences sont accessibles par leurs noms ou numéros d'accession à l'adresse http://cbi.labri.fr/Genolevures/index.php#.

La demande PCT WO 2005/118814 divulgue un ensemble de modifications génétiques permettant à la levure *Saccharomyces cerevisiae* de synthétiser des acides gras polyinsaturés.

Dans les levures, la dégradation des acides gras se produit par une bêta-oxydation, un processus en plusieurs étapes nécessitant quatre activités enzymatiques différentes.

Chez les levures, les enzymes sont essentiellement localisées dans les peroxysomes, contrairement aux mammifères chez qui elles sont localisées dans les mitochondries et les peroxysomes.

La mobilisation des lipides accumulés se produit au cours des trois phases distinctes :
(i) au cours de la phase exponentielle, les composés lipidiques stockés sont utilisés pour la synthèse des lipides membranaires afin de soutenir la croissance et la division cellulaire,
(ii) au cours de la phase stationnaire, lors de l'épuisement des nutriments, les acides gras libres sont libérés, plutôt lentement, à partir des triglycérides et soumis à la β- oxydation peroxysomale ;
(iii) en sortie de phase de carence, par exemple quand les cellules sortent de phase stationnaire et ré-entrent dans la phase de croissance végétative avec supplémentation en carbone, les dépôts lipidiques sont très rapidement dégradés en acides gras libres.

Le glycérol-3-phosphate (G3P), est un acteur crucial dans le métabolisme des triglycérides.

Il existe deux voies de synthèse pour le glycérol-3-phosphate.
- Dans la première, le glycérol-3-phosphate est dérivé du glycérol par l'intermédiaire de la glycerol kinase codée par le gène *GUT1*.
- Dans la deuxième voie, le glycérol-3-phosphate est directement synthétisée à partir de la dihydroxyacétone phosphate (DHAP). Cette réaction est catalysée par la glycérol-3-phosphate déshydrogénase (codée par le gène *GPD1*). Cette dernière réaction est réversible et la formation de DHAP à partir de glycérol-3-phosphate est catalysée par une deuxième isoforme de la glycérol-3-phosphate déshydrogénase, dont le gène est dénommé *GUT2.*

Les présents inventeurs ont précédemment montré que l'inactivation du gène *GUT2* entraine une accumulation accrue de lipides chez les levures, particulièrement chez *Y. lipolytica* (WO 2010/004141 ; Beopoulos et al., Appl Environ Microbiol., 74(24): 7779-7789, 2008). Le gène *GUT2* code l'isoforme Gut2p de la glycérol-3-phosphate déshydrogénase, laquelle catalyse la réaction d'oxydation du glycérol-3-phosphate en DHAP.

De façon surprenante et inattendue, la surexpression de la glycérol-3-phosphate déshydrogénase codée par le gène *GDP1* (SEQ ID NO : 1 ; SEQ ID NO : 2), qui est l'enzyme catalysant la réaction de synthèse de glycérol-3-phosphate à partir du DHAP, n'a aucun effet sur l'accumulation de lipides dans une souche qui est par ailleurs sauvage. De même, la surexpression de *GPD1* dans un mutant inactivé pour le gène *GUT2* n'a aucun effet sur l'accumulation de lipides dans les levures.

En revanche, les inventeurs ont montré qu'il est possible d'obtenir une accumulation de lipides en surexprimant le gène *GPD1* dans des levures chez lesquelles la bêta-oxydation des acides gras est déficiente.

Cette accumulation est bien plus importante que tout ce qui a été décrit précédemment dans l'art antérieur. En particulier, alors que les souches dans lesquelles *GUT2* est inactivé accumulent au plus 50 % de lipides quand elles sont cultivées en présence d'acide oléique (WO 2010/004141 ; Beopoulos et al., Appl Environ Microbiol., 74(24): 7779-7789, 2008), les souches qui sont déficientes dans la bêta-oxydation et qui surexpriment le gène *GPD1* accumulent, dans les mêmes conditions, plus de 60 % de lipides.

En outre, les souches présentant une déficience de la bêta-oxydation et surexprimant le gène *GPD1* présentent la particularité de lyser facilement, ce qui n'est pas le cas avec les souches de l'art antérieur. Cette propriété présente un intérêt certain d'un point de vue industriel, puisque facilitant la lyse des cellules pour récupérer les lipides produits.

Il est donc ici décrit une souche de levure chez laquelle la bêta-oxydation des acides gras est déficiente et qui surexprime le gène *GPD1,* ladite souche mutante étant capable d'accumuler des lipides.

On entend par le terme « levure » les souches de levure en général, c'est-à-dire que ce terme comprend, entre autres, *Saccharomyces cerevisiae*, *Saccharomyces sp*., *Hansenula polymorpha, Schizzosaccharomyces pombe, Yarrowia lipolytica, Pichia pastoris, Pichia finlandica, Pichia trehalophila, Pichia koclamae, Pichia membranaefaciens*, *Pichia minuta* (*Ogataea minuta, Pichia lindneri*), *Pichia opuntiae, Pichia thermotolerans, Pichia salictaria, Pichia guercuum, Pichia pijperi, Pichia stiptis, Pichia methanolica, Pichia sp., Kluyveromyces sp., Kluyveromyces lactis, Candida albicans.*

Les levures de l'invention sont les levures oléagineuses (Ratledge, in : Ratlege C, Wilkinson SG editors, Microbial lipids, Vol 2. London : Academic press 1988). Alors que la surexpression du gène *GPD1* conduit chez *S. cerevisiae,* dans n'importe quel fonds génétique, essentiellement à une accumulation de glycérol, elle résulte chez les levures oléagineuses déficientes pour la bêta-oxydation des acides gras en une accumulation de lipides.

La présente souche est donc préférentiellement une souche de levure oléagineuse chez laquelle la bêta-oxydation des acides gras est déficiente et qui surexprime le gène *GPD1,* ladite souche mutante étant capable d'accumuler des lipides.

Les levures oléagineuses les plus connues incluent les genres *Candida, Cryptoccocus, Rhodotorula, Rhizopus, Trichosporon, Lypomyces, Yarrowia.* Les levures particulièrement préférées au sens de l'invention comprennent *Yarrowia lipolytica, Rhodotura glutinis* et *Rhodosporidium torulides.* Une levure préférée au sens de la présente invention est *Yarrowia lipolytica.*

La bêta-oxydation est la voie de dégradation des acides gras. Elle fait intervenir 4 réactions successives au cours desquelles interviennent une acyl-CoA oxydase dont les 6 isoformes sont codée par les 6 gènes *POX*, une enzyme multifonctionnelle codée par le gène *MFE1* et une 3-oxoacyl-CoA thiolase codée par le gène *POT1.* Comme indiqué plus haut, la bêta-oxydation chez les levures se déroule exclusivement dans le peroxysome, un organite cytoplasmique dont la biogénèse est contrôlée par les gènes *PEX* (voir Tableau 2). Quand le peroxysome n'est pas correctement assemblé ou quand il n'est pas fonctionnel, les acides gras ne sont pas correctement dégradés (WO 2006/064131 ; Thevenieau et al., Fungal Genet Biol., 44(6): 531-42, 2007).

De façon générale, les mutations affectant la bêta-oxydation sont des mutations de perte de fonction qui entrainent une très forte diminution, voire une absence totale, de la bêta oxydation. Les mutations de perte de fonction peuvent correspondre à des mutations ponctuelles, à des insertions, à des délétions totales ou partielles, à des remplacements de gènes ou à toute autre cause moléculaire qui aboutit à une diminution substantielle de la bêta-oxydation.

Les souches de levure chez lesquelles la bêta-oxydation des acides gras est déficiente comprennent toutes les souches portant au moins une mutation de perte de fonction dans au moins un gène codant une enzyme directement impliquée dans la bêta-oxydation, mais aussi toutes les souches qui portent au moins une mutation de perte de fonction qui n'affecte la bêta-oxydation que de façon indirecte, notamment à travers la biogénèse ou la fonction des peroxysomes. Il est bien entendu que les souches décrites ici comprennent aussi toutes les souches portant des combinaisons des mutations décrites plus haut. Par exemple, entrent aussi dans le cadre de la présente description les souches qui portent au moins une mutation de perte de fonction affectant directement la bêta-oxydation et au moins une mutation de perte de fonction n'affectant la bêta-oxydation que de façon indirecte.

Les souches déficientes dans la bêta-oxydation des acides gras comprennent, selon un aspect préféré de l'invention, toute souche portant une mutation de perte de fonction dans un des gènes *PEX* listés dans le tableau 2. Selon un autre aspect préféré de l'invention, les souches déficientes dans la bêta-oxydation des acides gras comprennent les souches portant au moins une mutation de perte de fonction dans l'un des gènes suivants : *POX1, POX2, POX3, POX4, POX5, POX6, MFE1, POT1.* Plus préférentiellement, les souches selon l'invention comprennent au moins une mutation de perte de fonction dans l'un au moins des gènes *POX1, POX2, POX3, POX4, POX5* et *POX6.* De façon encore plus préférée, les souches selon l'invention comprennent des mutations dans chacun des gènes *POX1, POX2, POX3, POX4, POX5* et *POX6.*

Selon un mode particulier de réalisation, l'invention a donc pour objet une nouvelle souche de levure oléagineuse, plus particulièrement une souche de *Y. lipolytica,* mutante, qui surexprime le gène *GPD1* et qui comporte au moins une mutation de perte de fonction dans un des gènes responsable de la bêta-oxydation des acides gras, ladite souche de levure étant capable d'accumuler des lipides. Ladite souche de levure comporte au moins une mutation de perte de fonction dans au moins un des gènes choisis parmi les gènes *PEX, POX, MFE1* et *POT1.* De manière plus particulièrement préférée, les gènes *POX* sont partiellement (*POX2* à *POX5*) ou totalement (*POX1* à *POX6*) inactivés dans la souche mutante de l'invention, ladite souche mutante étant capable d'accumuler des lipides.

Outre les mutations de perte de fonction précitées, qui conduisent à une déficience de la bêta-oxydation, la présente souche de levure peut comporter une ou plusieurs mutations additionnelles dans au moins un gène codant une enzyme impliquée dans le métabolisme des acides gras. Cette(ces) mutation(s) additionnelle(s) peut(vent) avoir pour effet d'augmenter encore la capacité de la souche à accumuler des lipides. Alternativement, elle(s) peut(vent) altérer le profil des acides gras stockés. Ainsi, les présents inventeurs ont aussi montré que l'expression des gènes *TGL3* et *TGL4* est augmentée quand les gènes *POX* sont inactivés. Les gènes *TGL3* et *TGL4* codent des lipases impliquées dans la dégradation des acides gras (Kurat et al., J. Biol. Chem., 281 : 491-500, 2006). Sans vouloir être limité par la théorie, on peut penser que l'augmentation de l'expression de ces deux gènes sert sans doute à compenser l'absence d'une voie de bêta-oxydation fonctionnelle pour la dégradation des acides gras. L'invention a donc aussi pour objet une souche de levure, préférentiellement une souche de levure oléagineuse, plus particulièrement une souche de *Y. lipolytica,* mutante, déficiente pour la bêta-oxydation, déficiente pour les produits des gènes *TGL3* et *TGL4,* ladite souche surexprimant le gène *GPD1* et étant capable d'accumuler des lipides.

Selon un autre mode de réalisation préféré de l'invention, la souche de levure, oléagineuse, préférentiellement *Y. lipolytica,* mutante, déficiente pour la bêta-oxydation, ladite souche surexprimant le gène *GPD1* et étant capable d'accumuler des lipides, comporte en outre une mutation de perte de fonction dans le gène *GUT2.*

Il a aussi été montré que l'inactivation du gène *YALI0B10153g,* qui code une Δ12 désaturase d'acide gras, permet d'augmenter la proportion d'acides gras en C18 :1 (WO 2005/047485). C'est donc aussi un objet de la présente invention que de fournir une souche de levure, notamment de levure oléagineuse, préférentiellement de *Y*. *lipolytica,* mutante, déficiente pour la bêta-oxydation, qui surexprime le gène *GPD1* et est capable d'accumuler des lipides, et qui, en outre, comporte un gène *YALI0B10153g* inactivé.

Selon encore un autre aspect, la souche de levure oléagineuses, préférentiellement *Y. lipolytica,* mutante, déficiente pour la bêta-oxydation, qui surexprime le gène GPD1 et est capable d'accumuler des lipides, contient en outre un gène dont l'expression permet de modifier le profil en acide gras de ladite souche. Il a en effet été rapporté que l'expression ectopique de gènes codant certaines désaturases permet de modifier le profil en acides gras polyinsaturés dans une souche de levure et en particulier chez *Y. lipolytica.* Ainsi l'expression d'une Δ12 désaturase d'acide gras permet d'obtenir des acides gras en C18 :2 en plus grande quantité (WO 2005/047485). De la même façon, l'expression d'une Δ8 désaturase ou d'une Δ15 désaturase conduit à un changement du profil des acides gras chez *Y*. *lipolytica* (WO 2005/047480 ; WO 2006/012325). L'invention a donc aussi pour objet une souche de levure, notamment de *Y. lipolytica,* mutants, déficiente pour la bêta-oxydation, qui surexprime le gène GPD1 et est capable d'accumuler des lipides, et qui, en outre, exprime un gène codant pour une enzyme choisie parmi une Δ8 désaturase, une Δ12 désaturase et une Δ15 désaturase. De préférence, ladite enzyme est une Δ12 désaturase. Encore plus préférentiellement, le gène codant ladite Δ12 désaturase est le gène de *Y. lipolytica* dont le numéro d'accession est le *YALI0B10153g.*

La présente description a aussi pour objet un procédé d'obtention d'une souche de levure mutante, ladite souche mutante étant capable d'accumuler des lipides. Dans un mode préféré de réalisation, la levure est une levure oléagineuse. Dans un mode plus préféré de réalisation, la levure est *R. glutinis, R. tolurides* ou *Y. lipolytica.* Dans un mode encore plus préféré de réalisation, la levure est *Y. lipolytica.*

Ce procédé comporte au moins deux étapes, lesdites étapes pouvant être réalisées soit simultanément soit consécutivement. Si les deux étapes sont réalisées l'une après l'autre, l'ordre dans lequel elles sont réalisées importe peu. Ledit procédé comporte donc les étapes consistant à :
- inactiver au moins un gène contrôlant la bêta-oxydation et
- transformer la souche de levure par un polynucléotide permettant l'expression du gène *GPD1*

La première étape dudit procédé consiste donc à inactiver un gène responsable de la bêta-oxydation. Comme indiqué plus haut, ces gènes sont aussi bien les gènes *POX, MFE1, POT1* (Tableau 1) que les gènes *PEX* (Tableau 2).

L'art antérieur enseigne aussi différentes méthodes pouvant permettre l'obtention de souches de levure, particulièrement de levure oléagineuse, plus particulièrement *Y. lipolytica,* dans lesquelles un gène est inactivé.

Par exemple, on citera la méthode appelée POP IN/ POP OUT qui a été utilisée chez les levures, particulièrement chez *Y. lipolytica,* pour la délétion des gènes *LEU2, URA3* et *XPR2* comme il est décrit dans la revue de G. Barth et coll. : (*Yarrowia lipolytica,* in: Nonconventional Yeasts in Biotechnology A Handbook (Wolf, K., Ed.), Vol. 1 , 1996, pp. 313-388. Springer-Verlag). Elle consiste à intégrer un vecteur comprenant un gène d'intérêt délété au locus considéré, puis à sélectionner l'excision dudit vecteur et d'identifier un clone qui par recombinaison a éliminé le gène sauvage et conservé le gène muté.

Préférentiellement, on pourra utiliser une méthode conduisant à l'inactivation du gène d'intérêt.

Par « inactivation » ou « invalidation d'un gène d'intérêt » (les deux termes tels qu'utilisés dans la présente demande sont synonymes et ont donc la même signification), on entend toute méthode aboutissant à la non expression de la protéine native codée par ledit gène d'intérêt, par modification de l'enchaînement des nucléotides constituant ledit gène de telle sorte que quand bien même sa traduction serait effective, elle ne conduirait pas à l'expression de la protéine native codée par le gène d'intérêt sauvage.

Préférentiellement, on utilise une méthode conduisant à une extinction totale de l'expression du gène d'intérêt. Cela peut être réalisé par une délétion totale du gène d'intérêt, par une délétion partielle du gène d'intérêt, par l'insertion d'un ou plusieurs nucléotides dans ledit gène d'intérêt, ladite méthode utilisée rendant le gène d'intérêt non fonctionnel (gène d'intérêt inactivé ou invalidé), à tout le moins codant une protéine ne possédant pas les propriétés de la dite protéine native.

On obtient ainsi une souche de levure n'exprimant pas le gène d'intérêt que l'on nommera par la suite dans le présent texte "souche défective en gène d'intérêt".

On peut aussi utiliser la méthode SEP (Maftahi et al., Yeast, 12 : 859-868, 1996) qui a été adaptée chez *Y. lipolytica* pour la disruption successive des gènes *POX* (Wang et al., J. Bacteriol., 181: 5140-5148, 1999). Cette méthode est plus rapide, mais nécessite toujours l'utilisation d'un marqueur permettant une contre sélection. Avantageusement stilisera la méthode SEP/Cre développée par Fickers et al. (J. Microbiol. Methods, 55/3: 727-737, 2003) et décrite dans la demande internationale WO2006/064131. C'est une méthode rapide et qui ne nécessite pas l'utilisation d'un marqueur permettant une contre sélection.

Cette méthode consiste à :
1) sélectionner le gène d'intérêt que l'on veut déléter,
2) construire une cassette de disruption par PCR (« Polymerase Chain Reaction) ou par clonage,
3) introduire un marqueur de sélection contenant de part et d'autre des séquences de recombinaison (avantageusement des séquences *loxP* et/ou *loxR* ou dérivées) permettant une recombinaison entre elles pour l'élimination du marqueur (avantageusement une séquence de type *loxP* qui permet la recombinaison sous l'action de la recombinase Cre),
4) sélectionner les souches avec le gène d'intérêt délété (transformation et sélection des transformants) et de vérifier la délétion,
5) transformer avec un vecteur permettant l'expression de la recombinase (avantageusement la recombinase Cre qui permet la recombinaison des séquences *loxP*/*loxR* et l'élimination du marqueur)
6) isoler un clone présentant la délétion du gène d'intérêt et ayant perdu le plasmide d'expression de la recombinase.

La cassette d'insertion de l'étape 2 comprend un gène codant un marqueur de sélection (gène de sélection), ledit gène étant préférentiellement encadré par les régions promotrices et terminatrices du gène d'intérêt, de façon à permettre le remplacement complet du gène d'intérêt par recombinaison homologue. Selon un mode particulier de réalisation, le gène de sélection est en outre encadré par une ou des séquences de recombinaison, lesdites séquences de recombinaison permettant une recombinaison entre elles conduisant à l'élimination du gène codant le marqueur de sélection. Avantageusement la ou les séquence(s) de recombinaison, est (ou sont) une (ou des) séquence(s) *loxP* ou une (ou des) séquence(s) *loxR* ou (une ou des) séquence(s) dérivée(s) de ces séquences de recombinaison, ladite (lesdites) séquence(s) dérivées(s) ayant conservée(s) l'activité des séquences de recombinaison d'origine. Préférentiellement à cette étape, le gène codant le marqueur de sélection pourra être encadré par des séquences de type *loxP* qui, sous l'action de la recombinase Cre se recombinent entre elles en donnant naissance à un plasmide comprenant la séquence du gène codant ledit marqueur de sélection.

L'introduction de la cassette d'invalidation à l'étape 3 dans la souche de levure récipiendaire peut se faire par toute technique connue de la personne du métier. Comme indiqué plus haut, on se réfèrera pour cela à G. Barth *et al.* (*Yarrowia lipolytica.* in: Nonconventional Yeasts in Biotechnology A Handbook (Wolf, K., Ed.), Vol. 1, 1996, pp. 313-388. Springer-Verlag).

Les transformants exprimant le marqueur de sélection sont sélectionnés à l'étape 4. La présence du marqueur peut être vérifiée par toute méthode usuelle connue de la personne du métier, comme par exemple la PCR ou l'hybridation par Southern blot.

Dans l'étape 5, on introduit dans un transformant sélectionné dans l'étape précédente un plasmide permettant l'expression d'une recombinase. Préférentiellement, le plasmide sera porteur du gène de la recombinase Cre (Sauer, Mol. Cell. Biol., 7 : 2087-2096, 1987) qui permet la recombinaison des séquences *loxP*/*loxR* et l'élimination du marqueur. Cette technique est couramment utilisée par l'homme du métier cherchant à exciser une séquence intégrée spécifique (Hoess and Abremski, J. Mol. Biol., 181 : 351-362, 1984).

L'étape 6 est une étape standard de sélection d'un clone ayant excisé le gène de sélection et présentant donc un phénotype d'absence du marqueur de sélection.

Précisément, le procédé d'obtention d'une souche de levure, particulièrement d'une souche de levure oléagineuse, plus particulièrement d'une souche *Y. lipolytica,* mutante, n'exprimant pas un gène contrôlant la bêta-oxydation, est caractérisé en ce que
- dans une première étape, on construit une cassette d'invalidation comprenant les séquences promotrices et terminatrices dudit gène de levure, particulièrement de levure oléagineuse, plus particulièrement de *Y. lipolytica,* encadrant un gène codant un marqueur de sélection (gène de sélection), ledit gène de sélection étant lui-même encadré de part et d'autre de sa séquence par une (ou des) séquence(s) de recombinaison, lesdites séquences de recombinaison permettant une recombinaison entre elles conduisant à l'élimination dudit gène codant le marqueur de sélection ;
- dans une deuxième étape, on introduit ladite cassette d'invalidation obtenue en 1, dans une souche de levure, particulièrement une souche de levure oléagineuse, plus particulièrement *Y. lipolytica* ;
- dans une troisième étape, on sélectionne, parmi les souches de levure transformées à l'étape 2, une souche de levure, particulièrement une souche de levure oléagineuse, plus particulièrement une souche de *Y. lipolytica*, défective pour le gène d'intérêt, souche ayant introduit par double recombinaison (double cross-over) le gène marqueur en lieu et place dudit gène d'intérêt, conduisant ainsi à un gène inactivé ;
- dans une quatrième étape, on vérifie l'invalidation dudit gène dans ladite souche de levure sélectionnée à l'étape 3.

Selon une variante, le procédé peut en outre présenter 2 étapes supplémentaires à savoir :
- une cinquième étape au cours de laquelle on transforme ladite souche sélectionnée à l'étape 4 avec un vecteur permettant l'expression d'une recombinase afin d'obtenir l'élimination du gène exprimant le marqueur de sélection ;
- une sixième étape, au cours de laquelle on isole une souche de levure défective pour le gène, et n'exprimant plus le gène marqueur.

Le procédé d'inactivation d'un gène de la bêta-oxydation peut ensuite être répété de façon à inactiver un autre gène, si cela est nécessaire. La personne du métier sera ainsi à même d'inactiver autant de gènes que nécessaire, par simple répétition du procédé SEP d'inactivation de gène. Ladite personne peut ainsi construire les souches de levure mutantes décrites plus haut, qui comportent plusieurs gènes inactivés.

Selon l'invention, on pourra avantageusement utiliser une souche de levure qui ne réalisera pas la bêta-oxydation des lipides, par exemple une souche qui n'exprimera pas les gènes responsables de la bêta-oxydation des lipides comme les gènes *POX, MFE1* ou *POT1,* avantageusement une souche n'exprimant pas les gènes *POX,* à tout le moins les gènes *POX2, POX3, POX4* et *POX5,* préférentiellement les gènes *POX1, POX2, POX3, POX4, POX5* et *POX6,* comme par exemple les souches décrites dans la demande internationale WO 2006/064131 publiée le 22 juin 2006, préférentiellement les souches :
MTLY37 (Leu⁺, Ura⁺; Δ*pox5,* Δp*ox*2, Δ*pox3,* Δ*pox4 ::URA3*),
MTLY40 (Leu⁺, Ura⁻; Δ*pox5-PT,* Δ*pox2-PT,* Δ*pox3-PT,* Δ*pox4::ura3-41*),
MTLY64 (Leu⁻, Ura⁻; Hyg+; Δ*pox5,* Δ*pox2,* Δ*pox3,* Δ*pox4::ura3-41 , leu2 ::Hyg*)*,*
MTLY66 (Leu⁻, Ura⁻; Δ*pex5,* Δ*pox2,* Δ*pex3,* Δ*pox4::ura3-41,* Δ*leu2*),
MTLY82 (Leu⁻, Ura⁻; Hyg⁺; Δ*pox5,* Δ*pox2,* Δ*pox3,* Δ*pox4::ura3-41,* Δ*leu2, pox1 ::Hyg*),
MTLY86 Leu⁻, Ura⁻; Δ*pex5*; Δ*pex2,* Δ*pox3,* Δ*pox4::ura3-41,* Δ*leu2,* Δ*pox1*),
MTLY92 (Leu⁻, Ura⁻; Hyg+; Δ*pox5,* Δ*pex2,* Δ*pex3,* Δ*pox4::ura3-41,* Δ*leu2,* Δ*pox1, pox6::Hyg*),
MTLY95a (Leu⁻, Ura⁻; Δ*pex5,* Δ*pox2,* Δ*pex3,* Δ*pox4::ura3-41,* Δ*leu2,* Δ*pox1,* Δ*pox6*)

Dans un autre aspect du procédé, on pourra aussi utiliser une souche de levure telle que celles décrite dans la demande PCT WO 2010/004141, publiée le 14 janvier 2010. On pourra par exemple utiliser les souches :
JMY1351 (Leu⁻, Ura⁺, Δ*pox5,* Δ*pox2,* Δ*pox3,* Δ*pox4::ura3-41,* Δ*leu2,* Δ*pox1,* Δ*pox6,* Δ*gut2*)
JMY1393 (Leu⁻, Ura⁺, Δ*pox5,* Δ*pox2,* Δ*pox3,* Δ*pox4::ura3-41,* Δ*pox1,* Δ*pox6,* Δ*gut2*).

Dans encore un autre aspect du procédé, on pourra utiliser les souches décrites dans les publications Béopoulos et al. (Appl Environ Microbiol., 74(24): 7779-7789, 2008) et Wang et al (J. Bacteriol., 181: 5140-5148, 1999).

Dans une deuxième étape du procédé , le gène *GPD1* est surexprimé dans une des souches déficientes dans la bêta-oxydation obtenues à l'étape précédente.

Le gène *GPD1* code pour la glycérol-3-phosphate déshydrogénase catalysant la réaction de synthèse de glycérol-3-phosphate à partir du DHAP. Le gène *GPD1* peut être surexprimé de toutes les manières connues de la personne du métier.

Pour cela, chaque copie de la phase ouverte de lecture *GPD1* est placée sous le contrôle de séquences régulatrices appropriées. Lesdites séquences régulatrices comprennent des séquences promotrices, placées en amont (en 5') de la phase ouverte de lecture de *GPD1,* et des séquences terminatrices, placées en aval (en 3') de la phase ouverte de lecture de *GPD1.*

De préférence, les séquences promotrices et terminatrices utilisées appartiennent à des gènes différents de manière à minimiser les risques de recombinaison non désirée dans le génome de la souche de *Yarrowia.*

De telles séquences promotrices sont bien connues de l'homme du métier et peuvent correspondre notamment à des promoteurs inductibles ou constitutifs. A titre d'exemple de promoteurs utilisables dans le procédé selon l'invention, on peut citer notamment le promoteur d'un gène de *Y. lipolytica* qui est fortement réprimé par le glucose et qui est inductible par les acides gras ou les triglycérides tel que le promoteur *POX2* du gène de l'acyl CoA oxydase 2 de *Yarrowia lipolytica* et le promoteur du gène *LIP2* décrit dans la demande PCT WO 01/83773. On peut aussi utiliser le promoteur du gène *FBA1* du gène de la fructose-bisphosphate aldolase (US 2005/0130280), le promoteur du gène de la phosphoglycerate mutase *GPM* (WO 2006/0019297), le promoteur du gène *YAT1* du gène du transporteur de l'ammonium (US 2006/0094102 A1), le promoteur du gène *GPAT* du gène de la glycérol-3-phosphate O-acyltransferase (US 2006/0057690 A1), le promoteur du gène *TEF* (Muller et al., Yeast, 14 : 1267-1283, 1998 ; US 2001/6265185), le promoteur hybride *hp4d* (WO 96/41889) ou encore les promoteurs hybrides *XPR2* décrits dans Mazdak et al. (J Mol Microbiol Biotechnol., 2(2):207-16, 2000).

De telles séquences terminatrices sont également bien connues de l'homme du métier et on peut citer, à titre d'exemple de séquences terminatrices utilisables dans le procédé selon l'invention, la séquence terminatrice du gène *PGK1*, la séquence terminatrice du gène *LIP2* décrit dans la demande PCT WO 01 /83773.

La surexpression de *GPD1* peut être obtenue en remplaçant les séquences contrôlant l'expression de *GPD1* par des séquences régulatrices permettant une expression plus forte, telles que celles décrites ci-dessus. La personne du métier peut ainsi remplacer la copie du gène *GPD1* dans le génome, ainsi que ses séquences régulatrices propres, par transformation de la souche mutante de levure par un polynucléotide linéaire comprenant la phase ouverte de lecture de *GPD1* sous le contrôle de séquences régulatrices telles que celles décrites plus haut. Avantageusement, ledit polynucléotide est encadré par des séquences qui sont homologues de séquences situées de chaque côté du gène *GPD1* chromosomique. Dans la mesure où cet évènement de recombinaison est rare, des marqueurs de sélection sont insérés entre les séquences assurant la recombinaison afin de permettre, après transformation, d'isoler les cellules où l'intégration du fragment s'est produite par mise en évidence des marqueurs correspondant.

Avantageusement, la surexpression de *GPD1* est obtenue par l'introduction dans la souche de levure de l'invention de copies surnuméraires du gène *GPD1* sous le contrôle de séquences régulatrices telles que celles décrites plus haut. Lesdites copies supplémentaires de *GPD1* peuvent être portées par un vecteur épisomal, c'est-à-dire capable de se répliquer dans la levure.

Préférentiellement, elles sont portées par un vecteur intégratif, c'est-à-dire s'intégrant à un endroit donné dans le génome de la levure (Mazdak et al., J Biotechnol., 109(1-2): 63-81, 2004). Dans ce cas, le polynucléotide comprenant le gène *GPD1* sous le contrôle de régions régulatrices est intégré par intégration ciblée.

L'intégration ciblée d'un gène dans le génome d'une levure est une technique de biologie moléculaire fréquemment utilisée. Dans cette technique, un fragment d'ADN est cloné dans un vecteur intégratif, introduit dans la cellule à transformer, lequel fragment d'ADN s'intègre alors par recombinaison homologue dans une région ciblée du génome receveur (Orr-Weaver et al., Proc. Natl. Acad. Sci. USA, 78 : 6354-6358, 1981). De tels procédés de transformation sont bien connus de l'homme du métier et sont décrits, notamment, dans Ito et al. (J. Bacteriol., 153: 163-168, 1983) dans Klebe et al. (Gene, 25: 333-341, 1983) et dans Gysler et al. (Biotechn. Techn., 4: 285-290, 1990). Dans la mesure où cet évènement de recombinaison est rare, des marqueurs de sélection sont insérés entre les séquences assurant la recombinaison afin de permettre, après transformation, d'isoler les cellules où l'intégration du fragment s'est produite par mise en évidence des marqueurs correspondant.

Elles peuvent aussi être portées par des fragments de PCR dont les extrémités présentent de l'homologie avec un locus donné de la levure, permettant ainsi l'intégration desdites copies dans le génome de la levure par recombinaison homologue.

Toute méthode de transfert connue de l'art antérieur peut être utilisée pour introduire la cassette d'invalidation 1 dans la souche de levure. Préférentiellement on pourra utiliser la méthode à l'acétate de lithium et au polyéthylène glycol (Gaillardin et al., Curr. Genet., 11 : 369-375, 1987 ; Le Dall et al., Curr Genet, 26(1): 38- 44, 1994).

Il est ainsi possible d'utiliser toute méthode de sélection connue de l'art antérieur compatible avec le gène (ou les gènes) marqueur(s) utilisés, toute souche exprimant le gène marqueur choisi étant potentiellement une souche de levure détective en gène *GUT2, URA3* ou *LEU2.*

Les marqueurs de sélection permettant la complémentation d'une auxotrophie, également communément appelés marqueurs d'auxotrophie, sont bien connus de l'homme du métier.

Le marqueur de sélection *URA3* est bien connu de l'homme du métier. Plus spécifiquement, une souche de *Y. lipolytica* dont le gène *URA3* (ladite séquence est aussi accessible par le numéro d'accession *YALI0E26719g* à l'adresse «http://cbi.labri.fr/Genolevures/index.php#»), codant pour l'orotidine-5'-phosphate décarboxylase, est inactivé (par exemple par délétion), ne sera pas capable de pousser sur un milieu non supplémenté en uracile. L'intégration du marqueur de sélection *URA3* dans cette souche de *Y. lipolytica* permettra alors de restaurer la croissance de cette souche sur un milieu dépourvu d'uracile.

Le marqueur de sélection *LEU2* décrit notamment dans le brevet US 4,937,189 est également bien connu de l'homme du métier. Plus spécifiquement, une souche de *Y*. *lipolytica* dont le gène *LEU2* (*YALI0E26719g*), codant la β-isopropylmalate déshydrogénase, est inactivé (par exemple par délétion), ne sera pas capable de pousser sur un milieu non supplémenté en leucine. Comme précédemment. L'intégration du marqueur de sélection *LEU2* dans cette souche de *Y. lipolytica* permettra alors de restaurer la croissance de cette souche sur un milieu non supplémenté en leucine.

Le marqueur de sélection *ADE2* est également bien connu de l'homme du métier dans le domaine de la transformation de la levure. Une souche de *Yarrowia* dont le gène *ADE2* (*YALI0B23188g*), codant pour la phosphoribosylaminoimidazole carboxylase, est inactivé (par exemple par délétion), ne sera pas capable de pousser sur un milieu non supplémenté en adénine. Là encore, l'intégration du marqueur de sélection *ADE2* dans cette souche de *Y. lipolytica* permettra alors de restaurer la croissance de cette souche sur un milieu non supplémenté en adénine.

Il est aussi ici décrit l'utilisation d'une souche de levure mutante, particulièrement de levure oléagineuse, notamment de *Y. lipolytica,* pour la synthèse de lipides, particulièrement des acides gras libres et des triacylglycérols. Dans un aspect plus particulièrement préféré, une souche de levure, plus particulièrement de levure oléagineuse, notamment de *Y. lipolytica,* mutante, déficiente pour la bêta-oxydation et surexprimant *GPD1,* ainsi que décrit plus haut, est utilisée pour la synthèse d'acides gras libres et de triacylglycérols.

La présente invention a aussi pour objet un procédé de synthèse de lipide dans lequel :
- dans une première étape, on cultive une souche de levure selon l'invention dans un milieu approprié et
- dans une seconde étape on récolte les lipides produits par la culture de l'étape 1.

Outre les dispositions qui précèdent, la présente invention comprend également d'autres caractéristiques et avantages qui ressortiront des exemples et Figures qui suivent, et qui doivent être considérés comme illustrant l'invention sans en limiter la portée.

### LEGENDES DES FIGURES

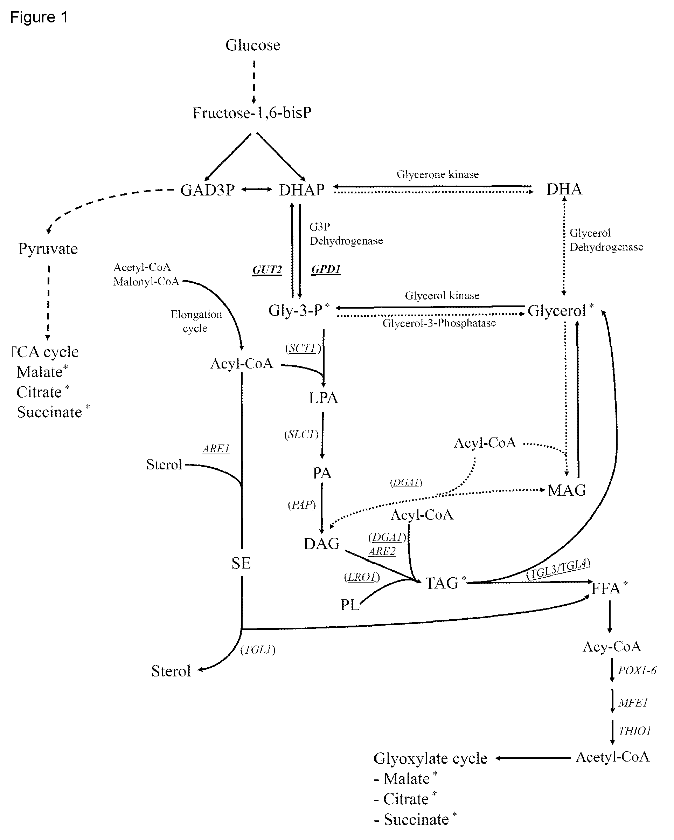

**Figure 1****.** Schéma des différentes voies de synthèse des acides gras, stockage et dégradation des lipides neutres et leur lien avec les cycles du glyoxylate et du TCA.
   Les flèches en pointillé indiquent des réactions enzymatiques hypothétiques. Les flèches avec tirets représentent des réactions simplifiées. Les protéines codées par les gènes entre parenthèses ont été montrées comme étant associées aux corps lipidiques (LB) chez *Y. lipolytica.* DAG, diacylglycerol; DHA, dihydroxyacetone; DHAP, dihydroxyacetone phosphate; FFA, free fatty acid; GAD3P, glyceraldehydes-3-phosphate; Gly-3P, glycerol-3-phosphate; LPA, lysophosphatidic acid; MAG, monoacylglycerol, PA, phosphatidic acid; PL, phospholipid; SE, steryl ester; TAG, triacylglycerol, TCA cycle, tricarboxylic acid cycle.

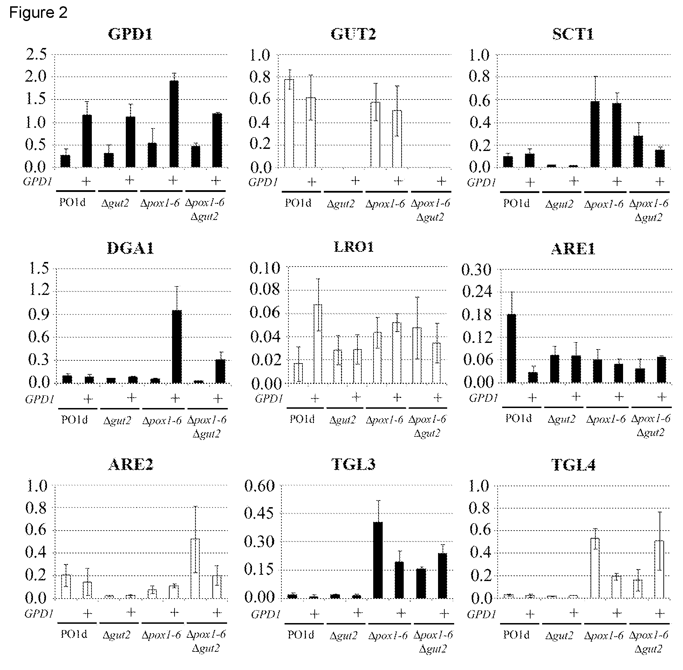

**Figure 2****.** Analyse de l'expression des gènes impliqués dans l'homéostase des triacylglycérols dans différentes souches de *Y. lipolytica.*
   L'expression des différents gènes a été mesurée par qRT-PCR en utilisant des amorces spécifiques de chacun des gènes et a été rapportée à la quantité de transcrits du gene *ACT1* codant pour l'actine (*YALI0D08272g*). Ces résultats correspondent à la moyenne ± déviation standard provenant de trois expériences indépendantes.
**Figure 3****.** Accumulation des lipides dans des souches différentes de *Y. lipolytica*
   Les levures ont été cultivées 24 h en YNBD2 (A) ou en YNBD0.5O3 (B). Les barres noires représentent les souches surexprimant *GPD1,* les barres blanches représentent les souches parentales. L'extraction des acides gras a été reproduite à partir de trois échantillons indépendants.
**Figure 4****.** Croissance de différentes souches en milieu YNBD0.5O3.
   La croissance des souches WT et mutantes listées dans le Tableau 1 a été suivie en fonction du temps. Ces résultats correspondent à la moyenne ± déviation standard provenant de trois expériences indépendantes.
**Figure 5****.** Phénotypes des LB dans différentes souches de *Y. lipolytica.*
   Les morphologies des LB des souches WT, *Δgut2, Δpox1-6, Δgut2Δpox1-6, WT GPD1*⁺, *Δgut2 GPD1⁺, Δpox1-6 GPD1*⁺ and *Δgut2Δpox1-6 GPD1*⁺ sont montrées. Les images en microscopie optique sont présentées sur les panneaux de gauche et les images correspondantes de microscopie en fluorescence sont présentées à droite. La fluorescence a été obtenue après coloration avec le colorant neutre vert LipidTox™. Les souches ont été cultivées pendant 24 h sur milieu YNBD0.5O3.

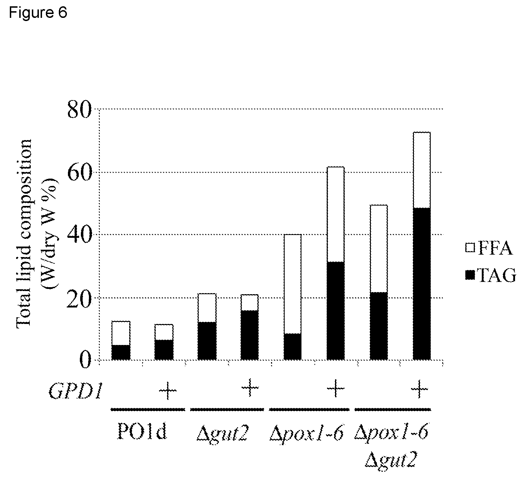

**Figure 6****.** Contenu en triacylglycérols et en acides gras libres en tant que fractions des lipides totaux.
   Ce graphe représente l'accumulation totale de lipides après 24 h de croissance en milieu YNBD0.5O3, en tant que pourcentage de la masse sèche des cellules. Les lipides ont été fractionnés en triacylglycérols et acides gras libres par extraction en phase solide et la quantification a été effectuée par chromatographie en phase gazeuse. L'extraction des acides gras a été reproduite à partir de deux échantillons indépendants ; un résultat représentatif des deux expériences est montré.

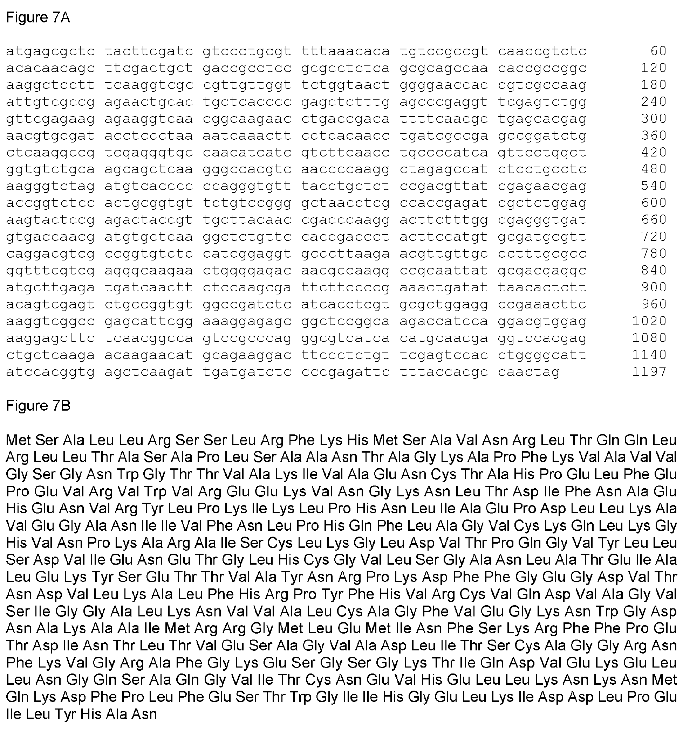

Figure 7. A. Séquence du gène *GPD1*⁺ ; B Séquence du polypeptide Gpd1.

### EXEMPLES

### Matériel et méthodes

### Souches de levure, conditions de croissance et de culture

Les souches de *Y. lipolytica* utilisées dans cette étude sont dérivées de la souche sauvage (wild-type ; WT) de *Y. lipolytica* W29 (ATCC20460) (Table 3). La souche auxotrophique Po1d (Leu⁻ Ura⁻) a été décrite par Barth and Gaillardin (1996. Yarrowia lipolytica, p. 313-388. In K. Wolf, K. D. Breunig, and G. Barth (ed.), Nonconventional yeasts in biotechnology, vol. 1. Springer-Verlag, Berlin, Germany.). La souche auxotrophique MTY94, dans laquelle les six gènes *POX* codant des acyl-CoA oxydases (Aox) ont été inactivatés, a été décrite précédemment (Beopoulos et al., Appl Environ Microbiol., 74(24): 7779-7789, 2008). La souche auxotrophique JMY1346, dans laquelle le gène *GUT2,* qui code la glycérol-3-phosphate déshydrogénase, a été inactivé, dérive de la souche JMY1202 (WO 2010/004141 ; Beopoulos et al., Appl Environ Microbiol., 74(24): 7779-7789, 2008). La souche auxotrophique JMY1367, dans laquelle les six gènes *POX* qui codent les acyl-CoA oxidases (Aox) et le gène *GUT2,* qui code la glycérol-3-phosphate déshydrogénase, ont été inactivés, dérive de la souche JMY1351 (Beopoulos et al., Appl Environ Microbiol., 74(24): 7779-7789, 2008). Pour obtenir JMY1346 et JMY1367, le marqueur *URA3* a été excisé de JMY1202 et de JMY1351, respectivement, ainsi que précédemment décrit (Beopoulos et al., Appl Environ Microbiol., 74(24): 7779-7789, 2008). Les souches utilisées dans cette étude sont listées dans le Tableau 3.

Les milieux et les conditions de culture pour *Escherichia coli* sont décrits dans Sambrook et al. (1989. Molecular cloning: a laboratory manual, 2nd ed. Cold Spring Harbor Laboratory Press, Cold Spring Harbor, NY), et ceux pour *Y. lipolytica* sont décrits dans Barth et Gaillardin (1996. Yarrowia lipolytica, p. 313-388. In K. Wolf, K. D. Breunig, and G. Barth (ed.), Nonconventional yeasts in biotechnology, vol. 1. Springer-Verlag, Berlin, Germany). Le milieu riche (YPD) et le milieu minimal avec du glucose (YNB) ont été préparés comme précédemment décrits (Mlickova' et al., Appl. Environ. Microbiol., 70: 3918-3924, 2004).

Le milieu minimum YNB contient 0,17 % de YNBww (milieu YNB sans acides aminés et sulfate d'ammonium ; Difco, Paris, France), 0,5 % de NH₄Cl, 0,1% d'extrait de levure (Bacto-DB) et 50 mM de tampon phosphate à pH 6,8. Ce milieu peut être supplémenté avec 0.2 % de casamino-acides (Difco, Paris, France) et/ou 0,1 g/L d'uracile.

Les milieux supplémentés en source de carbone sont :
- le milieu YNBD (glucose à 2%, Merck, Fontenay-sous-Bois Cedex, France) ;
- le milieu YNBG (glycérol à 2%, Merck);
- le milieu YNBDO (milieu supplémenté à 3% en acide oléique de Merck pur à 60%) ;
- le milieu YNBOu.p. (milieu supplémenté à 3% en acide oléique de Flucka pur à 98%) ;
- le milieu YNBO (YNBD0.5O3) utilisé pour suivre l'accumulation optimale et la remobilisation des acides gras est du milieu YNB supplémenté à 0,5% de glucose et 3% d'acide oléique ;
- le milieu YP₂D₄O₃ utilisé pour optimiser l'accumulation de lipides contient de l'extrait de levure (1%), de la protéose peptone (2%), du glucose (4%) et de l'acide oléique (3%). De l'uracile (0,1 g/L) et de la leucine (0,2 g/L) ont été ajoutés en cas de besoin.

Pour les milieux solides, 1,5 % d'agar a été ajouté.

L'acide oléique est émulsionné par sonication, en présence de 0,02% de Tween 40 (Mlickova' et al., Appl. Environ. Microbiol., 70: 3918-3924, 2004).

En règle générale, les cultures ont été réalisées comme suit : à partir d'une boite YPD, une première pré-culture est inoculée dans du milieu YPD (15 ml dans des Erlenmeyers de 50 ml à 170 tr / min, à 28°C pendant 6 h). Les cellules ont été utilisées pour inoculer une pré-culture en milieu YNBD (50 ml dans un Erlenmeyer de 500 ml à 170 tr / min, à 28°C, pendant une nuit).

Pour la culture, des cellules en croissance exponentielle ont été récoltées par centrifugation au lendemain de la pré-culture, lavées et resuspendues dans du milieu YNB frais à une densité optique à 600 nm de 0,5.

Pour déterminer la croissance des cellules, les cultures ont été centrifugées à 3000 g pendant 5 min, et le culot de cellules a été lavé deux fois avec des volumes égaux de solution SB (9 g / L de NaCl - 0,5% BSA). La biomasse produite a été déterminée en mesurant la densité optique à 600 nm et en estimant le poids sec des cellules après lyophilisation.

### Techniques générales de génétique

Les techniques générales de génétique moléculaire ont été utilisées comme décrit dans Sambrook et al. (1989. Molecular cloning: a laboratory manual, 2nd ed. Cold Spring Harbor Laboratory Press, Cold Spring Harbor, NY). Les enzymes de restriction utilisées proviennent de Eurogentec SA (Liège, Belgique).

La transformation des cellules de levure a été réalisée selon la méthode à l'acétate de lithium décrite dans Le Dall et al. (Curr Genet, 26(1): 38- 44, 1994). L'ADN génomique de levure (sauvage ou transformées) a été préparé tel que décrit par Querol et al. (Appl. Environ. Microbiol., 58(9) : 2948-2953, 1992). Les amplifications par PCR ont été réalisées sur un thermocycleur Eppendorf 2720, avec soit de l'ADN polymérase Taq (Promega, Madison, Wl, USA) soit de l'ADN polymérase *Pfu* (Stratagene, La Jolla, Californie). Le gène *GPD1* a été amplifié avec les amorces décrites dans le tableau 4. Les fragments de PCR ont été purifiés au moyen du kit de purification QIAGEN (Qiagen, Hilden, Allemagne) et les fragments d'ADN récupérés à partir de gels d'agarose en utilisant un kit QIAquick Gel Extraction (Qiagen, Hilden, Allemagne). L'ensemble de programmes Staden (Dear. et Staden, Nucleic Acid Res., 19 : 3907-3911, 1991) a été utilisé pour l'analyse des séquences.

### Préparation des ARN et quantification des transcrits

Les échantillons biologiques ont été congelés dans l'azote liquide et gardés à -80°C. Les ARN ont été extraits avec le kit RNeasy Mini Kit (Qiagen, Courtaboeuf, France) en suivant les instructions du fabricant. Pour les expériences de Q-PCR, l'ARN total de chaque échantillon a été traité à la Dnasel (Ambion). L'absence d'ADN génomique dans les préparations d'ARN a été vérifiée par PCR en utilisant les ARN totaux traités à la Dnase I comme matrice et la *Taq* (Promega, Madison, WI) comme enzyme. La qualité des ARN totaux a été contrôlée avec le Bioanalyzer Agitent 2100, les réactifs du kit Agitent RNA 6000 Nano et des puces à ARN. Les ARN totaux traités à la Dnase I (1 µg) ont été traités avec la reverse transcriptase Thermoscript RT (Invitrogen, Carlsbad, USA), en suivant les instructions du fabricant. Les expériences de Q-PCR ont été réalisées à l'aide du LightCycler 1.5 (Roche, Meylan, France) avec le kit LightCycler Fast Start DNA Master Sybr Green (Roche, Meylan, France), en suivant les instructions du fabricant. L'expression de chaque gène a été quantifiée avec la méthode de 2ΔCT, en calibrant avec *ACT1* qui code l'actine. La réaction d'amplification était la suivante: 95°C 8 min, 95°C 10 s, 60°C 6 sec, and 72°C 10 sec (45 cycles), 95°C 15 s, 60°C 15 s, and 95°C 15 s, 40°C 5 min. La mesure de l'expression de chaque gène par Q-PCR a été reproduite sur de trois échantillons indépendants. Les amorces utilisées pour la Q-PCR sont indiquées dans le Tableau 4.

### Microscopie par fluorescence

Pour la visualisation des corps lipidiques (LB), le fluorochrome LipidTOX™ Green (2.5 mg/ml en éthanol; Invitrogen) a été ajouté à une suspension de cellules (*A*₆₀₀ = 5) et incubé pendant 10 min à température ambiante. La microscopie a été réalisée avec un microscope à fluorescence, AXIO Imager M1 (Zeiss, Le Pecq, France) avec un objectif à immersion 100X. Le programme AxioVision Rel. 4.6 a été utilisé pour l'acquisition des images.

### Détermination des lipides

Les lipides d'une quantité de cellules supérieure à 5 mg ont été extraits soit par la procédure de Folch et al. (J Biol Chem, 226(1) : 497-509, 1957) pour l'analyse TLC ou directement convertis en leurs esters méthyliques en utilisant des cellules lyophilisées selon Browse et al., (Anal Biochem, 152(1): 141-145, 1986) pour l'analyse par chromatographie en phase gazeuse. La transméthylation complète a été vérifiée par une méthode au BF₃ dans le méthanol (Athenstaedt et al., J Bacteriol, 181(20) : 6441-6448, 1999) et sur plaques TLC.

L'analyse par chromatographie en phase gazeuse des esters méthyliques d'acides gras a été réalisée à l'aide d'un Varian 3900 équipé d'un détecteur à ionisation de flamme et une colonne Varian FactorFour vf-23ms, (3 pA à 260°C [30 m, 0,25, 0,25 µm]).

Les acides gras libres ont été identifiés par comparaison avec des esters méthyliques d'acides gras normes (Fatty Acid Methyl Ester, FAME, Cayman, Supelco, Sigma (France)) et quantifiés par la méthode de l'étalon interne en ajoutant 50 µg de C17:0 commercial (Sigma).

### Analyse des corps lipidiques

L'analyse des corps lipidiques a été faite en observant une culture cellulaire en croissance au microscope tel que décrit plus haut. Des objectifs de contraste de phase différentiel (Nomarski) ont été utilisés pour obtenir les images transmises.

### Analyse des classes de lipides

Les lipides totaux ont été fractionnés en triacylglycérols et acides gras libres pour la quantification des lipides en utilisant une colonne lsolute SPE Aminopropyl (IST, France, Paris, France) en suivant l'enseignement de Laffargue et al. (Plant Physiol Biochem, 45(3-4) : 250-257, 2007). Le conditionnement de la colonne a été réalisé 3 fois avec 3 ml d'hexane normal à débit normal. Un ml de l'ensemble des lipides extraits par la méthode de Folch et al. (J Biol Chem, 226(1) : 497-509, 1957) dans du CHCl₃ a été chargé dans la colonne et la fraction des lipides neutres a été recueillie.

L'élution totale des lipides neutres a été réalisée par lavage de la colonne 3 fois avec 3 ml de CHCl₃/isopropanol (2/1). La fraction des acides gras libres a été recueillie en lavant la colonne 3 fois avec 3 ml de ET₂O/acideacétique 2% avec un débit normal. Le solvant des fractions a été évaporé sous flux d'azote direct et la transméthylation a été suivie pour analyse par chromatographie en phase gazeuse (Laffargue et al., Plant Physiol Biochem, 45(3-4) : 250-257, 2007).

Des plaques TLC ont été utilisées pour les extractions de vérification. L'efficacité de la procédure a également été vérifiée par comparaison des profils de chromatographie en phase gazeuse des échantillons fractionnés ou non.

### Séparation des lipides par TLC

Des plaques TLC (silice G60, 20*20 cm, épaisseur 0,25 mm) (Merck, Allemagne) ont été utilisées. Les différentes classes de lipides ont été séparées en utilisant un système de solvant à double de développement : Système A (demi-plaque de migration): éther de pétrole/éther éthylique/ acide acétique: 20/20/0,8 (v/v/v); Système B (toute la plaque de migration): Ether de pétrole/Et₂O 49/1 (v/v). Une solution à 5% d'acide phosphomolybdique a été pulvérisée sur les plaques et les bandes de lipides ont été révélées au bout de 10 min à 105°C.

### Spectroscopie par RMN

Des extraits PCA (acide perchlorique) ont été préparés à partir de 1 g en poids sec de *Y. lipolytica* en suivant la méthode décrite dans Auber et al. (J. Cell Biol., 133: 1251-1263, 1996). Les valeurs sont exprimées en mg g⁻¹ de poids sec de levure. Les spectres ont été enregistrés sur un spectromètre Bruker RMN (AMX 400, wide bore; Bruker, Billerica, MA) équipé avec une sonde multinucléaire de 10 mm ou de 25 mm réglée à 161.9 ou 100.6 MHz pour les analyses de ³¹P- et ¹³C. La résonance du deutérium ²H₂O a été utilisée comme signal de référence. Pour les mesures, les acquisition de ¹³C -NMR et de ³¹P-NMR ont été réalisées comme décrit précédemment (Dulermo et al., New Phytol, 183 :1149-1162, 2009). Les solvants utilisés pour la RMN proviennent de Sigma-Aldrich et de Leman (Archamps, France).

### Résultats

### Construction of strains

Le gène *GPD1* de *Y. lipolytica* a été surexprimé dans des souches WT, *Δgut2, Δpox1-6* and *Δgut2Δpox1-6* de *Y. lipolytica.* Ces souches ont été transformées avec une cassette d'expression contenant *GPD1* sous le contrôle du promoteur constitutif *TEF* (Muller et al., Yeast, 14:1267-1283, 1998). Les transformants contenant la cassette d'expression ont été identifiés par PCR (data not shown). Il a été observé par RT-PCR quantitative que le gène *GPD1* était exprimé aux mêmes niveaux dans les différents transformants (Fig. 2). Dans les souches *GPD1*⁺, l'expression de *GPD1* est augmentée d'un facteur 5 à 6 par rapport au sauvage. Des résultats identiques ont été obtenus en analysant deux transformants de chaque souche *GPD1*⁺.

### La surexpression de GPD1 entraine une augmentation de la quantité de lipides cellulaires

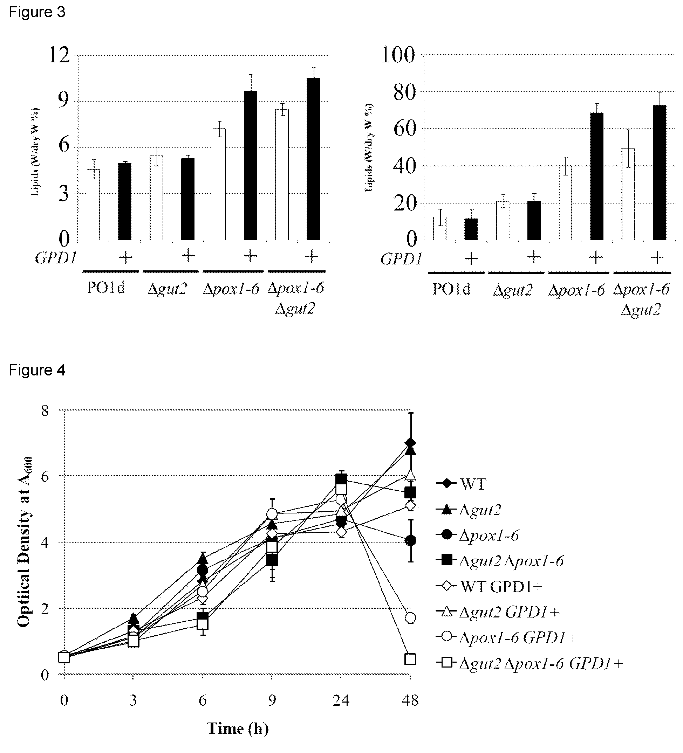

Tout d'abord, la capacité des différentes souches de synthétiser des lipides *de novo* a été étudiée après 24 h de culture en YNBD2 : à 24 h, la surexpression de *GPD1* n'a aucun effet dans le sauvage et dans *Δgut2,* alors que la quantité de lipides cellulaires est augmentée de 34 % et de 25% dans les souches *Δpox1-6 GPD1*⁺ et *Δgut2Δpox1-6 GPD*⁺, respectivement, par comparaison avec les souches parentales isogéniques (Fig. 3A).

Dans un second temps, la capacité d'accumulation des lipides des différentes souches a été analysée. Pour cela, les souches ont été cultivées en YNBD0.5O3. Alors que tous les transformants présentaient la même croissance que le sauvage en YNBD2 (data not shown), ce n'est pas le cas en YNBD0.5O3. Dans ce milieu particulier, la croissance de *Δgut2Δpox1-6 GPD1*⁺, et, dans une moindre mesure, de *Δpox1-6 GPD1*⁺ est dramatiquement affectée entre 24 et 48 h de culture, alors que ce n'est pas le cas pour les autres souches. En particulier, l'absorption de ces deux cultures chute brutalement entre 24 et 48 h, indiquant une lyse cellulaire (Fig. 4). La mort de ces cellules est probablement due à une trop forte accumulation de lipides. Pour cette raison, l'accumulation de lipides a été analysée dans les différents transformants après 24 h de culture en YNBD0.5O3 (Fig. 3B). Tout comme pour la synthèse *de novo* de lipides en YNBD2, l'accumulation de lipides est uniquement augmentée dans les souches *Δpox1-6 GPD1*⁺ et *Δgut2Δpox1-6 GPD1*⁺ de 70 % et 47 % par rapport à leurs souches parentales isogéniques, respectivement. L'accumulation des lipides représente plus de 65-75 % du poids sec de ces souches, c'est-à-dire 5 fois la quantité de lipides dans la souche sauvage à 24 h. La surexpression de *GPD1* n'a aucun effet sur le profil en acides gras des transformants (Table 5).

### Analyse microscopique de souches surexprimant GPD1

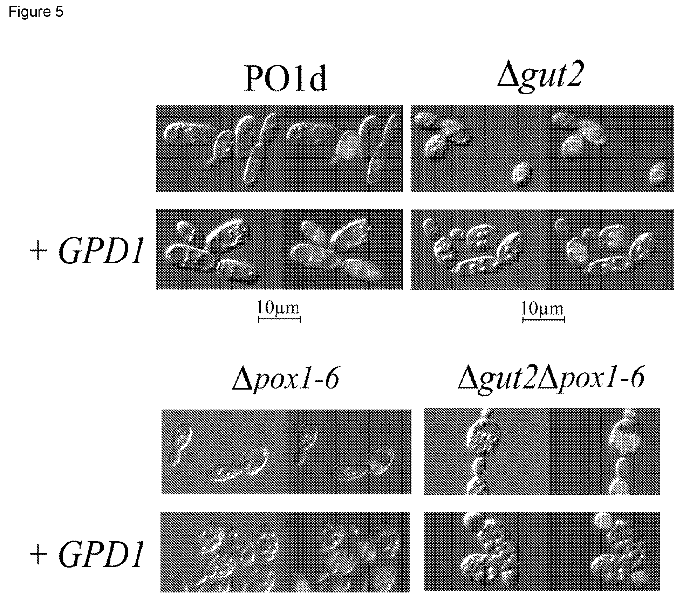

La surexpression de *GPD1* entraine une augmentation du contenu lipidique dans les souches *Δpox1-6* et *Δgut2Δpox1-6.* Chez S. *cerevisiae,* la plupart des lipides stockés sont des lipides neutres, c'est-à-dire des triacylglycérols et des esters de stérols. Chez *Y. lipolytica,* les lipides neutres sont stockés dans un compartiment spécialisé, le corps lipidique (LB) (Czabany et al., Biochim. Biophys. Acta, 1771: 299-309, 2007). Afin d'estimer l'effet de la surexpression de *GPD1* sur les corps lipidiques de *Y. lipolytica,* les cellules des différentes souches ont été analysées au microscope, après 24 h de croissance sur YNBD0.5O3. Les cellules *Δgut2, Δgut2 GPD1+, Δpox1-6, Δpox1-6 GPD1+, Δgut2Δpox1-6* and *Δgut2Δpox1-6 GPD1*⁺ montraient des corps lipidiques très importants (Fig. 5). Aucune différence n'a pu être détectée entre le WT et le WT *GPD1*⁺, ainsi qu'entre le *Δgut2* et le *Δgut2 GPD1*⁺*.* En revanche, les souches *Δpox1-6 GPD1*⁺ et *Δgut2Δpox1-6 GPD1*⁺ présentaient les plus gros corps lipidiques et les plus nombreux. La surexpression de *GPD1* non seulement entraine une augmentation de la quantité de lipides cellulaires mais semble aussi affecter le nombre et la taille des corps lipidiques chez *Y. lipolytica.*

### La surexpression de GPD1 entraine une augmentation de la fraction des triacylglycérols

Afin de quantifier la fraction des triacylglycérols dans les souches in *GPD1*⁺, les lipides neutres et les acides gras libres ont été fractionnés avec une colonne SPE Aminopropyl. Les fractions de triacylglycérols et d'acides gras libres ont été ensuite analysées par chromatographie en phase gazeuse.

La quantité de triacylglycérols a été analysée dans toutes les souches après 24 h de culture en YNBD0.5O3 (Fig. 6).

La délétion de *GUT2* et/ou la surexpression de *GPD1* conduisent à une augmentation de la fraction des triacylglycérols. Le niveau de triacylglycérols est augmenté d'un facteur 1,3, 2,4 and 3,2 dans les souches WT *GPD1*⁺, *Δgut2* et *Δgut2 GPD1*⁺, par rapport à la souche sauvage, respectivement. L'inactivation des gènes *POX1-6* a un petit effet sur le niveau de triacylglycérols, avec une augmentation d'un facteur 1,7 par comparaison avec la souche sauvage. En revanche, l'association des mutations *POX1-6* avec l'inactivation de *GUT2* et/ou la sureexpression de *GPD1* a un effet majeur sur la quantité de triacylglycérols, avec des niveaux de triacylglycérols dans les souches *Δgut2Δpox1-6, Δpox1-6 GPD1*⁺ et *Δgut2Δpox1-6 GPD1*⁺ atteignant 4, 6 et 10 fois respectivement le niveau observé dans la souche sauvage. Dans la souche *Δgut2Δpox1-6 GPD1*⁺ la fraction des triacylglycérols correspond à plus de 50% du poids sec.

### Analyses métaboliques des différentes souches par RMN

Les résultats obtenus montrent clairement que la surexpression de *GPD1* affecte dramatiquement la synthèse des triacylglycérols. Ceci pourrait être expliqué par une augmentation de la concentration du G3P. Afin de vérifier si l'inactivation de *GUT2* ou la surexpression de *GPD1* affecte la synthèse de G3P, les souches parentes et *GPD1*⁺ ont été cultivées sur YNBD0.5O3, et les métabolites ont été analysés par RMN. Les analyses des spectres de ³¹P indiquent que la concentration en G3P est augmentée de 1,5 à 5,6 fois, dans les souches où *GUT2* est inactivé ou dans les souches surexprimant *GPD1,* par rapport à la souche sauvage (Table 6). Quand *GUT2* est fonctionnel, la surexpression de *GPD1* n'a que peu d'effets sur le niveau de G3P. Par exemple, la concentration de G3P est augmentée de 50% seulement dans les souches WT *GPD1*⁺ et *Δpox1-6 GPD1*⁺ en comparaison des souches parentales. Le niveau de G3P est légèrement plus élevé dans la souche *Δpox1-6* comparée à la souche sauvage. La concentration de G3P est augmentée d'un facteur trois dans les souches *Δgut2* et *Δgut2Δpox1-6* par rapport à la souche sauvage. La concentration maximale de G3P est obtenue dans les souches *Δgut2 GPD1*⁺*.* De plus, la concentration G3P dans la souche *Δpox1-6 GPD1*⁺ et de façon surprenante dans la souche *Δgut2Δpox1-6 GPD1*⁺ est seulement deux fois la concentration de la sauvage. Il semble donc que la délétion de *GUT2* ou la surexpression de *GPD1* ont des conséquences différentes sur la synthèse de G3P.

Le G3P est un métabolite intermédiaire de la synthèse du glycérol. Alors que la concentration de G3P est augmentée dans les souches *Δgut2* et *GPD1*⁺, la concentration de glycérol est diminuée dans ces souches et encore plus dramatiquement dans les souches *Δgut2 GPD1*⁺, *Δpox1-6 GPD1*⁺, *Δgut2Δpox1-6* et *Δgut2Δpox1-6 GPD1*⁺.

Dans *Δgut2 GPD1*⁺, la souche accumulant les plus hauts niveaux de G3P, un doublement et un triplement des concentrations respectives de glycérol phosphorylcholine (GPC) et de glycérol phosphoryléthanolamine (GPE) a été observé. La RMN n'étant pas suffisamment sensible, il n'a pas été possible de détecter plus de trois intermédiaires des cycles du glyoxylate/TCA (malate, citrate et succinate) ; un forte diminution a été observée pour chacun de ces trois composés dans les souches où les gènes *POX* ou le gène *GUT2* était inactivé. De plus, la surexpression de *GPD1*⁺ dans ces souches accentue la disparition de ces composés.

### Modification de l'expression des gènes impliqués dans le métabolisme des triacylglycérides

Nous avons analysé par RT-PCR quantitative l'expression de *GUT2* et des gènes impliqués dans la synthèse des triacylglycérols, tels que *SCT1, DGA1, LRO1, ARE1* et *ARE2,* qui codent des acyltransférases (Beopoulos et al., Prog. Lipid Res, 48 : 375-387, 2009; Fig. 1). La surexpression de *GPD1* n'a aucun effet sur l'expression de *GUT2.* De même, la délétion de *GUT2* n'a aucun effet sur l'expression de *GPD1.* en revanche, un changement dans l'expression des gènes impliqués dans le métabolisme du G3P a un effet marqué sur *DGA1.* Dans les souches *Δpox1-6 GPD1*⁺ et *Δgut2Δpox1-6 GPD1*⁺, l'expression de *DGA1* est augmentée 15 et 10 fois, respectivement, par rapport aux souches parentales. De façon surprenante, la délétion des gènes *POX* entraine une forte augmentation de l'expression de *SCT1* tandis que la délétion de *GUT2* a un effet antagoniste et que la surexpression de *GPD1* n'a aucun effet. L'expression de *ARE1* est diminuée dans toutes les souches mutantes, alors que l'expression de *ARE2* est diminuée dans la souche *Δgut2* seulement. L'expression de *LRO1,* qui est faiblement exprimé, n'est pas modifiée dans les différentes souches.

L'accumulation des triacylglycérols ne dépend pas que des activités de synthèse. De fait, les processus de dégradation doivent avoir un rôle important rôle dans l'homéostasie des triacylglycérols. La première étape du catabolisme des triacylglycérols est catalysée par des lipases de triacylglycérols intracellulaires (Czabany et al., Biochim. Biophys. Acta, 1771 : 299-309, 2007; Fig. 1). La levure *Y*. *lipolytica* possède des lipases de triacylglycérols intracellulaires homologues des protéines Tgl3 et Tgl4 de S. *cerevisiae,* mais aucune homologue de Tgl5 (Beopoulos et al., Appl Environ Microbiol, 74 : 7779-7789, 2008). L'expression des gènes *TGL* (*TGL3* et *TGL4*) a été analysée par RT-PCR quantitative. Ces deux gènes ont des profils d'expression similaires. *TGL3* and *TGL4* sont fortement exprimés dans les souches dans lesquelles les gènes *POX* sont inactivés. Cependant, la délétion de *GUT2* ou la surexpression de *GPD1* a un effet négatif sur leur expression. Par exemple, l'expression de *TGL3* est augmentée 20 fois dans la souche *Δpox1-6* et seulement 7 fois dans la souche *Δpox1-6 Δgut2* par rapport à la souche sauvage. Dans leur ensemble, ces résultats montrent clairement que la modification du métabolisme du G3P ou des gènes *POX* affecte l'expression des gènes impliqués dans l'homéostasie des triacylglycérols.

**Tableau 1 : Gènes impliqués dans le métabolisme des acides gras chez les levures, particulièrement chez Y. lipolytica. Les séquences sont accessibles par leurs noms ou numéros d'accession à l'adresse « http://cbi.labri.fr/Genolevures/index.php# »**

| **Gène** | **Nom** | **N° EC** | **Fonction** |
|---|---|---|---|
| *GUT1* | *YALI0F00484g* | EC 2.7.1.30 | Glycerol kinase |
| *GPD1* | *YALI0B02948g* | EC 1.1.1.18 | Glycerol-3-phosphate dehydrogenase (NAD(+)) |
| *GUT2* | *YALI0813970g* | EC 1.1.99.5 | Glycerol-3-phosphate dehydrogenase |
| *SCT1* | *YALI0C00209g* | EC 2.3.1.15 | Glycerol-3-phosphate acyltransferase |
| *SLC1* | *YALI0E18964g* | EC 2.3.1.51 | 1-acyl-sn-glycerol- 3-phosphate acyltransferase |
| *DGA1* | *YALI0E32769g* | EC 2.3.1.20 | Diacylglycerol acyltransferase |
| *LRO1* | *YALI0E16797g* | EC 2.3.1.158 | Phospholipid :diacylglycerol acyltransferase |
| *TGL3* | *YALI0D17534g* | EC 3.1.1.3 | Triacylglycerol lipase |
| *TGL4* | *YALI0F10010g* | EC 3.1.1.3 | Triacylglycerol lipase |
| *ARE1* | *YALI0F06578g* | EC 2.3.1.26 | Acyl-CoA :sterol acyltransferase |
| *ARE2* | *YALI0D07986g* | EC 2.3.1.20 | Diacylglycerol acyltransferase |
| TGL1 | *YALI0E32035g* | EC 3.1.1.13 | Cholesterol esterase |
| *POX1* | *YALI0E32835g* | EC 6.2.1.3 | Acyl-coenzyme A oxidase |
| *POX2* | *YALI0F10857g* | EC 6.2.1.3 | Acyl-coenzyme A oxidase |
| *POX3* | *YALI0D24750g* | EC 6.2.1.3 | Acyl-coenzyme A oxidase |
| *POX4* | *YALI0E27654g* | EC 6.2.1.3 | Acyl-coenzyme A oxidase |
| *POX5* | *YALI0C23859g* | EC 6.2.1.3 | Acyl-coenzyme A oxidase |
| *POX6* | *YALI0E06567g* | EC 6.2.1.3 | Acyl-coenzyme A oxidase |
| *MFE1* | *YALI0E15378g* | EC 4.2.1.74 | Multi-functional beta oxidation protein |
| *POT1* | *YALI018568g* | EC 2.3.1.16 | Peroxisomal Oxoacyl Thiolase |

**Tableau 2 : Gènes impliqués dans le métabolisme des peroxysomes chez les levures, particulièrement chez Y. lipolytica. Les séquences sont accessibles par leurs noms ou numéros d'accession à l'adresse « http://cbi.labri.fr/Genolevures/index.php# »**

| **Gène** | **N° accession** | **N° accession** | **Fonction (ou source pour 3 gènes)** |
|---|---|---|---|
| | ***S. cerevisiae*** | ***Y. lipolytica*** | |
| *PEX1* | *YKL197c* | *YALI0C15356g* | AAA-peroxin |
| *PEX2* | *YJL210W* | *YALI0F01012g* | RING-finger peroxin which functions in peroxisomal matrix protein import |
| *PEX3* | *YDR329c* | *YALI0F22539g* | Peroxisomal membrane protein (PMP) |
| *PEX4* | *YGR133w* | *YALI0E04620g* | Peroxisomal ubiquitin conjugating enzyme |
| *PEX5* | *YDR244w* | *YALI0F28457g* | Peroxisomal membrane signal receptor |
| *PEX6* | *YNL329c* | *YALI0C18689g* | AAA-peroxin |
| *PEX7* | *YDR142c* | *YALI0F18480g* | Peroxisomal signal receptor |
| *PEX8* | *YGR077c* | / | Intraperoxisomal organizer of the peroxisomal import machinery |
| *PEX9* | / | *YALI0E14729g* | Peroxisomal intégral membrane protein |
| *PEX10* | *YDR265w* | *YALI0C01023g* | Peroxisomal membrane E3 ubiquitin ligase |
| *PEX11* | *YOL147c* | *YALI0C04092g* | Peroxisomal membrane protein |
| *PEX12* | *YMR026c* | *YALI0D26642g* | C3HC4-type RING-finger peroxisomal membrane peroxin |
| *PEX13* | *YLR191w* | *YALI0C05775g* | Integral peroxisomal membrane |
| *PEX14* | *YGL153w* | *YALI0E9405g* | Peroxisomal membrane peroxin |
| *PEX15* | *YOL044w* | / | Phosphorylated tail-anchored type II integral peroxisomal membrane protein |
| *PEX16* | / | *YALI0E16599g* | Intraperoxisomal peripheral membrane peroxin |
| *PEX17* | *YNL214w* | / | Peroxisomal membrane peroxin |
| *PEX18* | *YHR160c* | / | Peroxin |
| *PEX19* | *YDL065c* | *YALI0822660g* | Chaperone and import receptor |
| *PEX20* | / | *YALI0E06831g* | Peroxin |
| *PEX21* | *YGR239c* | / | Peroxin |
| *PEX22* | *YAL055w* | / | Putative peroxisomal membrane protein |
| *PEX23* | *PEX30 (YLR324w)* | *YALI0D27302g* | Integral peroxisomal membrane peroxin |
| | *PEX31* (*YGR004w*) | | |
| | *PEX32* (*YBR168w*) | | |
| *PEX25* | *YPL112c* | *YALI0D05005g* | Peripheral peroxisomal membrane peroxin |
| *PEX27* | *YOR193w* | / | Peripheral peroxisomal membrane protein |
| *PEX28* | *YHR150w* | *YALI0D11858g YALI0F19580g* | Peroxisomal integral membrane peroxin |
| *PEX29* | *YDR479c* | *YALI0F19580g* | Peroxisomal integral membrane peroxin |
| *PEX30* | *YLR324W* | *YALI0D27302g* | Peroxisomal integral membrane protein |
| *PEX31* | *YGR004W* | *YALI0D27302g* | Peroxisomal integral membrane protein |
| *PEX32* | *YBR168w* | *YALI0D27302g* | Peroxisomal integral membrane protein |

**Tableau 3 : Souches de E. coli et de Y. lipolytica utilisées.**

| **Strains (host strain)** | **Genotype or plasmid** | **Source or reference** |
|---|---|---|
| ***E. coli strains*** | | |
| Mach1T1 | *ΔrecA1398 endA1 tonA Φ80ΔlacM15 ΔlacX74 hsdR(r_{K}⁻ m_{K}*⁺*)* | Invitrogen |
| JME1128 | JMP 62 *URA3* Ex-pTEF-*GPD1* | This work |
| ***Y. lipolytica strains*** | | |
| W29 | *MAT*a WT | Aggelis et Sourdis, 1997 |
| PO1d | *MAT*a *ura3-302 leu2-270 xpr2-322* | Barth et |
| | | Gaillardin, 1996 |
| JMY1202 | *MAT***a** *ura3-302 leu2-270 xpr2-322* *Δgut2*::*URA3* | Beopoulos *et al.,* |
| JMY1233 | *MAT**a** ura3-302 xpr2-322 Δleu2 Δpox1-6* | Beopoulos et al., |
| JMY1346 | *MAT***a** *ura3-302 leu2-270 xpr2-322 Δgut2* | Beopoulos et al., |
| JMY1351 | *MAT***a** *ura3-302 xpr2-322 Δleu2* *Δpox1-6 Δgut2*::*URA3* | Beopoulos et al., |
| JMY1367 | *MAT**a** ura3-302 xpr2-322 Δleu2 Δpox1-6 Δgut2* | Beopoulos et al., |
| JMY1748 | *MAT**a** ura3-302 leu2-270 xpr2-322* + *pTEF-GPD1-URA3* | This work |
| JMY1789 | *MAT**a** ura3-302 xpr2-322 Δleu2* *Δpox1-6 Δgut2* + *pTEF-GPD1-URA3* | This work |
| JMY1796 | *MAT**a** ura3-302 leu2-270 xpr2-322* *Δgut2* + *pTEF-GPD1-URA3* | This work |
| JMY1972 | *MAT**a** ura3-302 xpr2-322 Δleu2* *Δpox1-6* + pTEF-*GPD1-URA3* | This work |

**Tableau 4 : Amorces utilisées pour la construction des vecteurs et la Q-PCR. Pour chaque paire d'amorce, la première est l'amorce sens (« forward »), la deuxième l'amorce antisens (« reverse »).**

| **Vecteur** | **Gène** | **Amorces pour construction de vecteur** |
|---|---|---|
| JME1128 | *GPD1* | SEQ ID NO: 3 |
| | | GCGGATCCCACAATGAGCGCTCTACTTCGATCGTCCC |
| | | SEQ ID NO: 4 |
| | | GCGCCTAGGCTAGTTGGCGTGGTAAAGAATCTCGGG |

| **Gènes** | **N° Accession** | **Amorces pour analyses de Q-PCR** |
|---|---|---|
| | | SEQ ID NO: 5 |
| | | TCCAGGCCGTCCTCTCCC |
| *ACT* | | SEQ ID NO: 6 |
| | | GGCCAGCCATATCGAGTCGCA |
| | | SEQ ID NO: 7 |
| *ARE1* | YALI0F06578g | TCCTCAAGCGACACGTCTA |
| | | SEQ ID NO: 8 |
| | | CAGCAACAGCAGGTATCC |
| | | SEQ ID NO: 9 |
| *ARE2* | YALI0D07986g | TTCTCATCTTCCAGTACGCCTA |
| | | SEQ ID NO: 10 |
| | | GGCAATAAGATTGAGACCGTT |
| | | SEQ ID NO: 11 |
| *DGA1* | YALI0E32769g | TGTACCGATTCCAGCAGT |
| | | SEQ ID NO: 12 |
| | | GGTGTGGGAGATAAGGCAA |
| | | SEQ ID NO: 13 |
| *GPD1* | YALI0B02948g | CGAGACTACCGTTGCTTAC |
| | | SEQ ID NO: 14 |
| | | CAACAACGTTCTTAAGGGC |
| | | SEQ ID NO: 15 |
| *GUT2* | YALI0F00484g | TGTGTGGAACCTCGACTACAA |
| | | SEQ ID NO: 16 |
| | | CATCCAGAAGTAGGGAAGC |
| | | SEQ ID NO: 17 |
| *LRO1* | YALI0E16797g | CTCCGCCGACTTCTTTATG |
| | | SEQ ID NO: 18 |
| | | GAAGTATCCGTCTCGGTG |
| | | SEQ ID NO: 19 |
| *SCT1* | YALI0C00209g | GAGATTGTGTCCGACACT |
| | | SEQ ID NO: 20 |
| | | TTTGTCGAATACGGATCGGT |
| | | SEQ ID NO: 21 |
| *TGL3* | YALI0D17534g | CAAGATTGTCTCTCCCTACG |
| | | SEQ ID NO: 22 |
| | | GTACTCGGATCAGGTTCAC |
| | | SEQ ID NO: 23 |
| *TGL4* | YALI0F10010g | GTTCGACAAGGAGCCTATT |
| | | SEQ ID NO: 24 |
| | | GGTCAGATGCGGATGATAAAG |

**Tableau 5 : Profils des acides gras des souches sauvage et mutantes. Les souches ont été cultivées en YNB avec 0,5 % glucose et 3 % acide oléique (70 % pur). Les valeurs indiquées correspondent à la moyenne de trois expériences différentes après 24 h de culture. La déviation standard était < 10 % des valeurs indiquées.**

| | PO1d | PO1d *GPD1+* | *Δgut2* | *Δgut2 GPD1+* | *Δpox1-6* | *Δpox1-6 GPD1+* | *Δgut2 Δpox1-6* | *Δgut2 Δpox1-6 GPD1+* |
|---|---|---|---|---|---|---|---|---|
| C16:0 | 6,0 | 5,1 | 5,0 | 4,9 | 4,0 | 4,1 | 2,6 | 5,3 |
| C16:1(n-9) | 1,1 | 0,9 | 13,7 | 14,4 | 0,3 | 0,4 | 0,2 | 0,4 |
| C16:1 (n-7) | 4,3 | 6,0 | 6,5 | 6,5 | 3,8 | 5,6 | 3,2 | 8,1 |
| C18:0 | 0,6 | 0,5 | 0,3 | 0,5 | 0,7 | 0,6 | 0,7 | 0,8 |
| C18:1(n-9) | 57,5 | 56,9 | 48,1 | 46,1 | 68,1 | 62,8 | 60,1 | 59,7 |
| C18:2(n-6) | 24,8 | 21,4 | 22,0 | 22,2 | 14,1 | 18,3 | 16,2 | 15,8 |
| Total | 94,3 | 90,9 | 95,6 | 94,6 | 91,0 | 91,8 | 83,0 | 90,1 |

**Tableau 6 : Profil des métabolites par analyse par RMN. Les métabolites ont été identifiés et quantifiés sur une série d'expériences, en utilisant le maléate et le méthyl-phosphonate comme standard internes. Deux expériences indépendantes ont été réalisées à partir de deux cultures séparées et ont donné des résultats similaires. Un résultat représentatif est présenté. G3P, glycerol-3-phosphate, GPC, glycérol phosphorylcholine, GPE, glycérol phosphoryléthanolamine.**

| | Metabolites^{a} | | | | | | | |
|---|---|---|---|---|---|---|---|---|
| | PO1d | PO1d *GPD1*⁺ | *Δgut2* | *Δgut2 GPD1*⁺ | *Δpox1-6* | *Δpox1-6 GPD1*⁺ | *Δgut2 Δpox1-6* | *Δgut2 Δpox1-6 GPD1*⁺ |
| ¹³C metabolites (mmoles/g of dry mass) | | | | | | | | |
| Glycerol | 8,5 | 4,2 | 5,2 | 2 | 4,4 | 3 | 0,8 | 1,5 |
| | | | | | | | | |
| Malate | 17,6 | 26,1 | 16,8 | 8 | 7,1 | 2,5 | 4 | 2,2 |
| Citrate | 2 | 1,2 | 1,3 | 0,8 | 0,7 | 0,5 | 0,5 | 0,7 |
| Succinate | 15,2 | 17,6 | 9,6 | 7,8 | 7,4 | 4,1 | 3,2 | 2,7 |

| ³¹P metabolites (µmoles/g of dry mass) | | | | | | | | |
|---|---|---|---|---|---|---|---|---|
| G3P | 5 | 7.7 | 14.4 | 28.5 | 7 | 10.3 | 14.6 | 11.7 |
| GPC | 67 | 50 | 80 | 112 | 69 | 66 | 75 | 52 |
| GPE | 10 | 7 | 11.1 | 37.4 | 7.7 | 6.4 | 10.8 | 7.8 |

## Revendications

1. Souche de levure oléagineuse mutante qui surexprime le gène *GPD1* par rapport à la souche sauvage et qui comporte au moins une mutation de perte de fonction dans au moins un gène choisi parmi les gènes *PEX,* les gènes *POX*, le gène *MFE1* et le gène *POT1,* ladite souche mutante étant capable d'accumuler des lipides.

2. La souche de levure oléagineuse de la revendication 1, **caractérisée en ce que** la levure est *Yarrowia lipolytica.*

3. La souche de levure oléagineuse de l'une quelconque des revendications 1 ou 2, **caractérisée en ce qu'**elle comporte au moins une mutation de perte de fonction dans au moins un des gènes *POX1, POX2, POX3, POX4, POX5* et *POX6.*

4. La souche de levure oléagineuse de l'une quelconque des revendications précédentes, **caractérisée en ce qu'**elle comporte une mutation de perte de fonction dans chacun des gènes *POX1, POX2, POX3, POX4, POX5* et *POX6.*

5. La souche de levure oléagineuse de l'une quelconque des revendications précédentes, **caractérisée en ce qu'**elle est en outre déficiente dans chacun des produits des gènes *TGL3* et *TGL4.*

6. La souche de levure oléagineuse de l'une quelconque des revendications précédentes,, **caractérisée en ce que** qu'elle comporte en outre un gène *GUT2* inactivé.

7. La souche de levure oléagineuse de l'une quelconque des revendications précédentes, **caractérisée en ce qu'**elle comporte un gène *YALI0B10153g* inactivé.

8. La souche de levure oléagineuse de l'une quelconque des revendications 1-6, **caractérisée en ce qu'**elle exprime un gène codant pour une enzyme choisie parmi une Δ8 désaturase, une Δ12 désaturase et une Δ15 désaturase.

9. La souche de levure oléagineuse de la revendication 8, **caractérisée en ce que** ledit gène code pour une Δ12 désaturase.

10. La souche de levure oléagineuse de la revendication 9, **caractérisée en ce que** ledit gène est le gène *YALI0B10153g* de *Yarrowia lipolytica.*

11. Procédé d'obtention d'une souche de levure oléagineuse selon l'une quelconque des revendications précédentes, comportant les étapes consistant à :
(a) inactiver au moins un gène choisi parmi les gènes *PEX,* les gènes *POX*, le gène *MFE1* et le gène *POT1* et
(b) transformer dans la souche de levure oléagineuse un polynucléotide permettant l'expression du gène *GPD1.*

12. Procédé selon la revendication 11 **caractérisé en ce que** la souche de levure oléagineuse est une souche de *Yarrowia lipolytica.*

13. Procédé selon l'une quelconque des revendications 11 et 12 **caractérisé en ce que** l'étape (a) comprend les étapes de :
1) sélectionner le gène d'intérêt que l'on veut inactiver parmi les gènes *PEX,* les gènes *POX*, le gène *MFE1* et le gène *POT1,*
2) construire une cassette de disruption,
3) introduire un marqueur de sélection contenant de part et d'autre des séquences de recombinaison permettant une recombinaison entre elles pour l'élimination du marqueur,
4) sélectionner les souches avec le gène d'intérêt délété et de vérifier la délétion,
5) transformer avec un vecteur permettant l'expression de la recombinase et
6) isoler un clone présentant la délétion du gène d'intérêt et ayant perdu le plasmide d'expression de la recombinase.

14. Procédé selon la revendication 13 **caractérisé en ce que** les séquences de recombinaison sont de séquences *loxP* et/ou *loxR* et que la recombinase est la recombinase Cre.

15. Procédé selon l'une quelconque des revendications 11 à 14, **caractérisé en ce que** l'étape (a) est répétée de façon à inactiver un autre gène.

16. Procédé selon l'une quelconque des revendications 11 à 15, **caractérisé en ce que** le gène *GPD1* de l'étape (b) est sous le contrôle de séquences promotrices et de séquences terminatrices.

17. Procédé selon l'une quelconque des revendications 11à 16, **caractérisé en ce que** la séquence promotrice est choisie parmi le promoteur *POX2,* le promoteur *LIP2,* le promoteur *FBA,* le promoteur *GPM,* le promoteur *YAT1,* le promoteur *GPAT,* le promoteur *TEF,* le promoteur hybride *hp4d* et des promoteurs hybrides *XPR2.*

18. Procédé selon l'une quelconque des revendications 11à 17, **caractérisé en ce que** la séquence promotrice est le promoteur *TEF1.*

19. Procédé selon l'une quelconque des revendications 11à 18, **caractérisé en ce que** la séquence terminatrice est choisie parmi la séquence terminatrice du gène *PGK1* et la séquence terminatrice du gène *LIP2.*

20. Utilisation d'une souche de levure oléagineuse mutante selon l'une des revendications 1 à 10 pour la synthèse de lipides, particulièrement des acides gras libres et des triacylglycérols.

21. Procédé de synthèse de lipide comportant des étapes:
(1) de culture d'une souche de levure oléagineuse selon l'une quelconque des revendications 1 à 10 dans un milieu approprié et
(2) de récolte les lipides produits par la culture de l'étape 1.

## Patentansprüche

1. Stamm einer mutierenden ölhaltigen Hefe, der das Gen *GPD1.* mit Bezug auf den wilden Stamm überexprimiert, und der mindestens eine Funktionsverlustmutation in mindestens einem Gen umfasst, ausgewählt aus den Genen *PEX,* den Genen *POX,* dem Gen *MFE1* und dem Gen *POT1,* wobei der mutierende Stamm dazu in der Lage ist, Lipide zu speichern.

2. Stamm einer ölhaltigen Hefe nach Anspruch 1, **dadurch gekennzeichnet, dass** die Hefe *Yarrowia lipolytica* ist.

3. Stamm einer ölhaltigen Hefe nach einem der Ansprüche 1 oder 2, **dadurch gekennzeichnet, dass** er mindestens eine Funktionsverlustmutation in mindestens einem der Gene *POX1, POX2, POX3, POX4, POX5* und *POX6* umfasst.

4. Stamm einer ölhaltigen Hefe nach einem der vorhergehenden Ansprüche, **dadurch gekennzeichnet, dass** er mindestens eine Funktionsverlustmutation in jedem der Gene *POX1, POX2, POX3, POX4, POX5* und POX6 umfasst.

5. Stamm einer ölhaltigen Hefe nach einem der vorhergehenden Ansprüche, **dadurch gekennzeichnet, dass** er außerdem in jedem der Produkte die Gene *TGL3* und *TGL4* nicht aufweist.

6. Stamm einer ölhaltigen Hefe nach einem der vorhergehenden Ansprüche, **dadurch gekennzeichnet, dass** er außerdem ein inaktiviertes Gen *GUT2* umfasst.

7. Stamm einer ölhaltigen Hefe nach einem der vorhergehenden Ansprüche, **dadurch gekennzeichnet, dass** er ein inaktiviertes Gen *YALI0B10153g* umfasst.

8. Stamm einer ölhaltigen Hefe nach einem der Ansprüche 1 - 6, **dadurch gekennzeichnet, dass** er ein Gen umfasst, das ein Enzym kodiert, ausgewählt aus einer Δ8 Desaturase, einer Δ12 Desaturase und eine Δ15 Desaturase.

9. Stamm einer ölhaltigen Hefe nach Anspruch 8, **dadurch gekennzeichnet, dass** das Gen eine A12 Desaturase codiert.

10. Stamm einer ölhaltigen Hefe nach Anspruch 9, **dadurch gekennzeichnet, dass** das Gen das Gen *YALI0B10153g* von *Yarrowia lipolytica* ist.

11. Verfahren zum Erhalt eines Stamms einer ölhaltigen Hefe nach einem der vorhergehenden Ansprüche, umfassend die Schritte, die aus Folgendem bestehen:
a) Inaktivieren von mindestens einem Gen, ausgewählt aus den Genen *PEX,* den Genen *POX,* dem Gen *MFE1* und dem Gen *POT1,*
b) Umwandeln, im Stamm einer ölhaltigen Hefe, eines Polynukleotid, das die Expression des Gens *GPD1* ermöglicht.

12. Verfahren nach Anspruch 11, **dadurch gekennzeichnet, dass** der Stamm einer ölhaltigen Hefe ein Stamm von *Yarrowia lipolytica* ist.

13. Verfahren nach einem der Ansprüche 11 und 12, **dadurch gekennzeichnet, dass** der Schritt (a) die folgenden Schritte umfasst:
1) Auswählen des bestimmten Gens, das inaktiviert werden soll, aus den Genen *PEX,* den Genen *POX,* dem Gen *MFE1* und dem Gen *POT1,*
2) Konstruieren einer Unterbrechungskassette,
3) Einführen eines Auswahlmarkers, der auf beiden Seiten Rekombinationssequenzen enthält, die eine Rekombination untereinander zur Entfernung des Markers ermöglichen,
4) Auswählen der Stämme mit dem bestimmten deletierten Gen und Überprüfen der Entfernung,
5) Umwandeln mit einem Vektor, der die Expression der Rekombinase ermöglicht, und
6) Isolieren eines Klons, der die Deletion des bestimmten Gens darstellt und das Plasmid zur Expression der Rekombinase verloren hat.

14. Verfahren nach Anspruch 13, **dadurch gekennzeichnet, dass** die Rekombinationssequenzen die Sequenzen *loxP* und/oder *loxR* sind, und dass die Rekombinase die Rekombinase Cre ist.

15. Verfahren nach einem der Ansprüche 11 bis 14, **dadurch gekennzeichnet, dass** der Schritt (a) wiederholt wird, um ein weiteres Gen zu inaktivieren.

16. Verfahren nach einem der Ansprüche 11 bis 15, **dadurch gekennzeichnet, dass** das Gen *GPD1* von Schritt (b) unter der Kontrolle von Promotorsequenzen und Terminatorsequenzen ist.

17. Verfahren nach einem der Ansprüche 11 bis 16, **dadurch gekennzeichnet, dass** die Promotersequenz ausgewählt ist aus dem Promoter *POX2,* dem Promoter *LIP2,* dem Promoter *FBA,* dem Promoter *GPM,* dem Promoter *YAT1,* dem Promoter *GPAT,* dem Promoter *TEF,* dem hybriden Promoter *hp4d* und den hybriden Promotoren *XPR2.*

18. Verfahren nach einem der Ansprüche 11 bis 17, **dadurch gekennzeichnet, dass** die Promotorsequenz der Promotor *TEF1* ist.

19. Verfahren nach einem der Ansprüche 11 bis 18, **dadurch gekennzeichnet, dass** die Terminatorsequenz ausgewählt ist aus der Terminatorsequenz von Gen *PGK1* und der Terminatorsequenz von Gen *LIP2*

20. Verwendung eines Stamms einer mutierenden ölhaltigen Hefe ach einem der Ansprüche 1 bis 10 für die Synthese von Lipiden, insbesondere der freien Fettsäuren und der Triacylglycerole.

21. Verfahren zur Lipidsynthese, umfassend die Schritte:
1) des Kultivierens eines Stamms einer ölhaltigen Hefe nach einem der Ansprüche 1 bis 10 in einem geeigneten Medium und
2) des Erntens der Lipide, hergestellt durch die Kultur von Schritt 1.

## Claims

1. Oleaginous yeast strain which overexpresses the *GPD1.* gene compared with a wild strain and comprises at least one loss-of-function mutation in at least one gene chosen from the *PEX* genes, the *POX* genes, the *MFE1* gene and the *POT1* gene, said mutant strain being capable of accumulating lipids.

2. The oleaginous yeast strain of claim 1, **characterised in that** the yeast is *Yarrowia lipolytica.*

3. The oleaginous yeast strain of one of claims 1 or 2, **characterised in that** it comprises at least one loss-of-function mutation in at least one of the *POX1, POX2, POX3, POX4, POX5* and *POX6* genes.

4. The oleaginous yeast strain of any of the preceding claims, **characterised in that** it comprises a loss-of-function mutation in each of the *POX1, POX2, POX3, POX4, POX5* and *POX6* genes.

5. The oleaginous yeast strain of any of the preceding claims, **characterised in that** it is also deficient in each of the products of the *TGL3* and *TGL4* genes.

6. The oleaginous yeast strain of any of the preceding claims, **characterised in that** it also comprises an inactivated *GUT2* gene.

7. The oleaginous yeast strain of any of the preceding claims, **characterised in that** it comprises an inactivated *YALI0B10153g* gene.

8. The oleaginous yeast strain of any of the claims 1-6, **characterised in that** it expresses a gene coding for an enzyme chosen from a Δ8 desaturase, a Δ12 desaturase and a Δ15 desaturase.

9. The oleaginous yeast strain of claim 8, **characterised in that** said gene codes for a Δ12 desaturase.

10. The oleaginous yeast strain of claim 9, **characterised in that** said gene is the *YALI0B10153g* gene of *Yarrowia lipolytica.*

11. Method for obtaining an oleaginous yeast strain of any of the preceding claims, comprising the steps consisting of:
(a) inactivating at least one gene chosen from the *PEX* genes, the *POX* genes, the *MFE1* gene and the *POT1* gene, and
(b) transforming, in the oleaginous yeast strain, a polynucleotide allowing expression of the *GPD1* gene.

12. Method according to claim 11, **characterised in that** the oleaginous yeast strain is a strain of *Yarrowia lipolytica.*

13. Method according to either of claims 11 and 12, **characterised in that** step (a) comprises the steps of:
1) selecting the gene of interest that it is wished to inactivate from the *PEX* genes, the *POX* genes, the *MFE1* gene and the *POT1* gene,
2) constructing a disruption cassette,
3) introducing a selection marker containing on either side recombination sequences allowing recombination between them in order to eliminate the marker,
4) selecting the strains with the gene of interest deleted and verifying the deletion,
5) transforming with a vector allowing expression of the recombinase, and
6) isolating a clone exhibiting the deletion of the gene of interest and having lost the expression plasmid of the recombinase.

14. Method according to claim 13, **characterised in that** the recombination sequences are *loxP* and/or *loxR* sequences and **in that** the recombinase is the Cre recombinase.

15. Method according to any of claims 11 to 14, **characterised in that** step (a) is repeated so as to inactivate another gene.

16. Method according to any of claims 11 to 15, **characterised in that** the *GPD1* gene of step (b) is under the control of promoter sequences and terminater sequences.

17. Method according to any of claims 11 to 16, **characterised in that** the promoter sequence is chosen from the *POX2* promoter, the *LIP2* promoter, the *FBA* promoter, the *GPM* promoter, the *YAT1* promoter, the *GPAT* promoter, the *TEF* promoter, the *hp4d* hybrid promoter, and the *XPR2* hybrid promoters.

18. Method according to any of claims 11 to 17, **characterised in that** the promoter sequence is the *TEF1* promoter.

19. Method according to any of claims 11 to 18, **characterised in that** the terminater sequence is chosen from the terminater sequences of the *PGK1* gene and the terminater sequence of the *LIP2* gene.

20. Use of a mutant oleaginous yeast strain according to one of claims 1 to 10 for synthesising lipids, in particular free fatty acids and triacylglycerols.

21. A method of synthesizing lipids comprising steps of:
(1) growing an oleaginous yeast strain according to any of claims 1 to 10 in a suitable medium, and
(2) harvesting the lipids produced by the culture of step 1.


### SEQUENCE LISTING

<110> INSTITUT NATIONAL DE LA RECHERCHE AGRONOMIQUE DULERMO, THIERRY NICAUD, JEAN MARC
<120> OPTIMISATION DE LA SYNTHESE ET DE L'ACCUMULATION DE LIPIDES
<130> 358602D28616
<150> FR 1055331
   <151> 2010-07-01
<160> 24
<170> PatentIn version 3.5
<210> 1
   <211> 1197
   <212> DNA
   <213> Yarrowia lipolytica
<400> 1
   atgagcgctc tacttcgatc gtccctgcgt tttaaacaca tgtccgccgt caaccgtctc      60
   acacaacagc ttcgactgct gaccgcctcc gcgcctctca gcgcagccaa caccgccggc     120
   aaggctcctt tcaaggtcgc cgttgttggt tctggtaact ggggaaccac cgtcgccaag     180
   attgtcgccg agaactgcac tgctcacccc gagctctttg agcccgaggt tcgagtctgg     240
   gttcgagaag agaaggtcaa cggcaagaac ctgaccgaca ttttcaacgc tgagcacgag     300
   aacgtgcgat acctccctaa aatcaaactt cctcacaacc tgatcgccga gccggatctg     360
   ctcaaggccg tcgagggtgc caacatcatc gtcttcaacc tgccccatca gttcctggct     420
   ggtgtctgca agcagctcaa gggccacgtc aaccccaagg ctagagccat ctcctgcctc     480
   aagggtctag atgtcacccc ccagggtgtt tacctgctct ccgacgttat cgagaacgag     540
   accggtctcc actgcggtgt tctgtccggg gctaacctcg ccaccgagat cgctctggag     600
   aagtactccg agactaccgt tgcttacaac cgacccaagg acttctttgg cgagggtgat     660
   gtgaccaacg atgtgctcaa ggctctgttc caccgaccct acttccatgt gcgatgcgtt     720
   caggacgtcg ccggtgtctc catcggaggt gcccttaaga acgttgttgc cctttgcgcc     780
   ggtttcgtcg agggcaagaa ctggggagac aacgccaagg ccgcaattat gcgacgaggc     840
   atgcttgaga tgatcaactt ctccaagcga ttcttccccg aaactgatat taacactctt     900
   acagtcgagt ctgccggtgt ggccgatctc atcacctcgt gcgctggagg ccgaaacttc     960
   aaggtcggcc gagcattcgg aaaggagagc ggctccggca agaccatcca ggacgtggag    1020
   aaggagcttc tcaacggcca gtccgcccag ggcgtcatca catgcaacga ggtccacgag    1080
   ctgctcaaga acaagaacat gcagaaggac ttccctctgt tcgagtccac ctggggcatt    1140
   atccacggtg agctcaagat tgatgatctc cccgagattc tttaccacgc caactag       1197
<210> 2
   <211> 398
   <212> PRT
   <213> Yarrowia lipolytica
<400> 2
   Met Ser Ala Leu Leu Arg Ser Ser Leu Arg Phe Lys His Met Ser Ala
   1               5                   10                  15
   Val Asn Arg Leu Thr Gln Gln Leu Arg Leu Leu Thr Ala Ser Ala Pro
               20                  25                  30
   Leu Ser Ala Ala Asn Thr Ala Gly Lys Ala Pro Phe Lys Val Ala Val
           35                  40                  45
   Val Gly Ser Gly Asn Trp Gly Thr Thr Val Ala Lys Ile Val Ala Glu
       50                  55                  60
   Asn Cys Thr Ala His Pro Glu Leu Phe Glu Pro Glu Val Arg Val Trp
   65                  70                  75                  80
   Val Arg Glu Glu Lys Val Asn Gly Lys Asn Leu Thr Asp Ile Phe Asn
                   85                  90                  95
   Ala Glu His Glu Asn Val Arg Tyr Leu Pro Lys Ile Lys Leu Pro His
               100                 105                 110
   Asn Leu Ile Ala Glu Pro Asp Leu Leu Lys Ala Val Glu Gly Ala Asn
           115                 120                 125
   Ile Ile Val Phe Asn Leu Pro His Gln Phe Leu Ala Gly Val Cys Lys
       130                 135                 140
   Gln Leu Lys Gly His Val Asn Pro Lys Ala Arg Ala Ile Ser Cys Leu
   145                 150                 155                 160
   Lys Gly Leu Asp Val Thr Pro Gln Gly Val Tyr Leu Leu Ser Asp Val
                   165                 170                 175
   Ile Glu Asn Glu Thr Gly Leu His Cys Gly Val Leu Ser Gly Ala Asn
               180                 185                 190
   Leu Ala Thr Glu Ile Ala Leu Glu Lys Tyr Ser Glu Thr Thr Val Ala
           195                 200                 205
   Tyr Asn Arg Pro Lys Asp Phe Phe Gly Glu Gly Asp Val Thr Asn Asp
       210                 215                 220
   Val Leu Lys Ala Leu Phe His Arg Pro Tyr Phe His Val Arg Cys Val
   225                 230                 235                 240
   Gln Asp Val Ala Gly Val Ser Ile Gly Gly Ala Leu Lys Asn Val Val
                   245                 250                 255
   Ala Leu Cys Ala Gly Phe Val Glu Gly Lys Asn Trp Gly Asp Asn Ala
               260                 265                 270
   Lys Ala Ala Ile Met Arg Arg Gly Met Leu Glu Met Ile Asn Phe Ser
           275                 280                 285
   Lys Arg Phe Phe Pro Glu Thr Asp Ile Asn Thr Leu Thr Val Glu Ser
       290                 295                 300
   Ala Gly Val Ala Asp Leu Ile Thr Ser Cys Ala Gly Gly Arg Asn Phe
   305                 310                 315                 320
   Lys Val Gly Arg Ala Phe Gly Lys Glu Ser Gly Ser Gly Lys Thr Ile
                   325                 330                 335
   Gln Asp Val Glu Lys Glu Leu Leu Asn Gly Gln Ser Ala Gln Gly Val
               340                 345                 350
   Ile Thr Cys Asn Glu Val His Glu Leu Leu Lys Asn Lys Asn Met Gln
           355                 360                 365
   Lys Asp Phe Pro Leu Phe Glu Ser Thr Trp Gly Ile Ile His Gly Glu
       370                 375                 380
   Leu Lys Ile Asp Asp Leu Pro Glu Ile Leu Tyr His Ala Asn
   385                 390                 395
<210> 3
   <211> 37
   <212> DNA
   <213> Artificial Sequence
<220>
   <223> Oligonucleotide
<400> 3
   gcggatccca caatgagcgc tctacttcga tcgtccc 37
<210> 4
   <211> 36
   <212> DNA
   <213> Artificial Sequence
<220>
   <223> Oligonucleotide
<400> 4
   gcgcctaggc tagttggcgt ggtaaagaat ctcggg 36
<210> 5
   <211> 18
   <212> DNA
   <213> Artificial Sequence
<220>
   <223> Oligonucleotide
<400> 5
   tccaggccgt cctctccc 18
<210> 6
   <211> 21
   <212> DNA
   <213> Artificial Sequence
<220>
   <223> Oligonucleotide
<400> 6
   ggccagccat atcgagtcgc a 21
<210> 7
   <211> 19
   <212> DNA
   <213> Artificial Sequence
<220>
   <223> Oligonucleotide
<400> 7
   tcctcaagcg acacgtcta 19
<210> 8
   <211> 18
   <212> DNA
   <213> Artificial Sequence
<220>
   <223> Oligonucleotide
<400> 8
   cagcaacagc aggtatcc 18
<210> 9
   <211> 22
   <212> DNA
   <213> Artificial Sequence
<220>
   <223> Oligonucleotide
<400> 9
   ttctcatctt ccagtacgcc ta 22
<210> 10
   <211> 21
   <212> DNA
   <213> Artificial Sequence
<220>
   <223> Oligonucleotide
<400> 10
   ggcaataaga ttgagaccgt t 21
<210> 11
   <211> 18
   <212> DNA
   <213> Artificial Sequence
<220>
   <223> Oligonucleotide
<400> 11
   tgtaccgatt ccagcagt 18
<210> 12
   <211> 19
   <212> DNA
   <213> Artificial Sequence
<220>
   <223> Oligonucleotide
<400> 12
   ggtgtgggag ataaggcaa 19
<210> 13
   <211> 19
   <212> DNA
   <213> Artificial Sequence
<220>
   <223> Oligonucleotide
<400> 13
   cgagactacc gttgcttac 19
<210> 14
   <211> 19
   <212> DNA
   <213> Artificial Sequence
<220>
   <223> Oligonucleotide
<400> 14
   caacaacgtt cttaagggc 19
<210> 15
   <211> 21
   <212> DNA
   <213> Artificial Sequence
<220>
   <223> Oligonucleotide
<400> 15
   tgtgtggaac ctcgactaca a 21
<210> 16
   <211> 19
   <212> DNA
   <213> Artificial Sequence
<220>
   <223> Oligonucleotide
<400> 16
   catccagaag tagggaagc 19
<210> 17
   <211> 19
   <212> DNA
   <213> Artificial Sequence
<220>
   <223> Oligonucleotide
<400> 17
   ctccgccgac ttctttatg 19
<210> 18
   <211> 18
   <212> DNA
   <213> Artificial Sequence
<220>
   <223> Oligonucleotide
<400> 18
   gaagtatccg tctcggtg 18
<210> 19
   <211> 18
   <212> DNA
   <213> Artificial Sequence
<220>
   <223> Oligonucleotide
<400> 19
   gagattgtgt ccgacact 18
<210> 20
   <211> 20
   <212> DNA
   <213> Artificial Sequence
<220>
   <223> Oligonucleotide
<400> 20
   tttgtcgaat acggatcggt 20
<210> 21
   <211> 20
   <212> DNA
   <213> Artificial Sequence
<220>
   <223> Oligonucleotide
<400> 21
   caagattgtc tctccctacg 20
<210> 22
   <211> 19
   <212> DNA
   <213> Artificial Sequence
<220>
   <223> Oligonucleotide
<400> 22
   gtactcggat caggttcac 19
<210> 23
   <211> 19
   <212> DNA
   <213> Artificial Sequence
<220>
   <223> Oligonucleotide
<400> 23
   gttcgacaag gagcctatt 19
<210> 24
   <211> 21
   <212> DNA
   <213> Artificial Sequence
<220>
   <223> Oligonucleotide
<400> 24
   ggtcagatgc ggatgataaa g 21